# EUROPEAN PATENT SPECIFICATION

(11) **EP 2 046 811 B1**
(45) Date of publication and mention of the grant of the patent: **03.08.2011**
(21) Application number: 07768941.2
(22) Date of filing: 28.06.2007
(51) Int. Cl.: C07K 2/00, C07K 14/435

(54) **Antimicrobial peptides**
Antimikrobielle Peptide
Peptides antimicrobiens

(30) Priority: 30.06.2006 NO 20063056
(43) Date of publication of application: 15.04.2009
(73) Proprietor: University Of Tromsø, 9037 Tormsø (NO)
(72) Inventor: STENSVÅG, Klara, N-9014 Tromsø (NO); HAUG, Tor, N-9100 Kvaløysletta (NO); LI, Chun, N-9012 Tromsø (NO); STYRVOLD, Olaf B., N-9017 Tromsø (NO); BLENCKE, Hans-Matti, 9100 Kvaløysletta (NO); PAULSEN, Victoria, N-9006 Tromsø (NO)
(74) Representative: Lillegraven, Rita
(86) International application number: PCT/NO2007/000241
(87) International publication number: WO 2008/002153

(56) References cited:
- HAUG TOR ET AL: "Antibacterial activity in Strongylocentrotus droebachiensis (Echinoidea), Cucumaria frondosa (Holothuroidea), and Asterias rubens (Asteroidea)." JOURNAL OF INVERTEBRATE PATHOLOGY, vol. 81, no. 2, October 2002 (2002-10), pages 94-102, XP002465550 ISSN: 0022-2011 cited in the application
- GROSS PAUL S ET AL: "Echinoderm immunity and the evolution of the complement system" DEVELOPMENTAL AND COMPARATIVE IMMUNOLOGY, vol. 23, no. 4-5, June 1999 (1999-06), pages 429-442, XP002465552 ISSN: 0145-305X
- DATABASE BIOSIS [Online] BIOSCIENCES INFORMATION SERVICE, PHILADELPHIA, PA, US; 2004, TERWILLIGER DAVID ET AL: "Unexpected genetic diversity in a putative antimicrobial peptide from a sea urchin" XP002465551 Database accession no. PREV200400285320 & FASEB JOURNAL, vol. 18, no. 4-5, 2004, page ABST. 777.4, FASEB MEETING ON EXPERIMENTAL BIOLOGY: TRANSLATING THE GENOME; WASHINGTON, DISTRICT OF COLUMBIA, USA; APRIL 17-21, 2004 ISSN: 0892-6638

### Sequence Listing

<110> University of Tromsø
   Stensvaag, Klara
   Blencke, Hans-Matti
   Paulsen, Victoria
   Li, chun
   styrvold, olaf Bay
   Haug, Tor
<120> Novel Polypeptides
<130> No20063056
<140> No20063056
   <141> 2006-06-30
<150> No20063056
   <151> 2006-06-30
<160> 45
<170> PatentIn version 3.1
<210> 1
   <211> 30
   <212> PRT
   <213> Strongylocentrotus droebachiensis
<220>
   <221> MISC_FEATURE
   <222> (2)..(2)
   <223> Unknown amino acid is marked "xaa" and located in position 2.
<400> 1
   Gly Xaa Phe Lys Lys Thr Phe His Lys Val Ser His Ala Val Lys Ser
   1               5                   10                  15
   Gly Ile His Ala Gly Gln Arg Gly Cys Ser Ala Leu Gly Phe
               20                  25                  30
<210> 2
   <211> 12
   <212> PRT
   <213> Strongylocentrotus droebachiensis
<400> 2
   Asp Leu Arg Gly Ala Cys Ala Ala Ala His Ala Leu
   1               5                   10
<210> 3
   <211> 33
   <212> PRT
   <213> Strongylocentrotus droebachiensis
<220>
   <221> MISC_FEATURE
   <222> (1)..(33)
   <223> Unknown amino acids are marked "xaa" and located in position 2 an d 28.
<400> 3
   Gly Xaa Gly Ser Lys Phe Lys Lys Ala Phe His Lys Val Ser His Val
   1 5 10 15
   Val Arg Gln Gly Ile His Ala Gly Gln Ser Val Xaa Ser Gly Val Gly
   20 25 30
   Leu
<210> 4
   <211> 12
   <212> PRT
   <213> Strongylocentrotus droebachiensis
<220>
   <221> MIS_FEATURE
   <222> (1)..(12)
   <223> Unknown amino acid is marked "Xaa" and located in position 6.
<400> 4
   Asp Leu Arg Ser Ile Xaa Ala Gly Ala His Ala Leu
   1 5 10
<210> 5
   <211> 30
   <212> PRT
   <213> Strongylocentrotus droebachiensis
<220>
   <221> MISC_FEATURE
   <222> (1)..(30)
   <223> unknown amino acids are marked "Xaa" and located in position 2, 1 5, 16, 25, 28 and 30.
<400> 5
   Ser Xaa Phe Ser Arg Thr Val His Asn Val Gly Asn Ala Val Xaa Xaa
   1 5 10 15
   Gly Ile His Ala Gly Gln Gly Val Xaa Ser Gly Xaa Gly Xaa
   20 25 30
<210> 6
   <211> 12
   <212> PRT
   <213> Strongylocentrotus droebachiensis
<220>
   <221> MIST_FEATURE
   <222> (1)..(12)
   <223> Unknown amino acid is marked "Xaa" and located in position 6.
<400> 6
   Asp Leu Arg Ala Ile Xaa Ala Gly Ala His Ala Leu
   1               5                   10
<210> 7
   <211> 30
   <212> PRT
   <213> Strongylocentrotus droebachiensis
<220>
   <221> MISC_FEATURE
   <222> (2)..(2)
   <223> unknown amino acid is marked "Xaa" and located in position 2.
<400> 7
   Ser Xaa Phe Ser Arg Thr Val His Asn Val Gly Asn Ala Val Arg Lys
   1               5                   10                  15
   Gly Ile His Ala Gly Gln Gly Val Cys Ser Gly Leu Gly Leu
               20                  25                  30
<210> 8
   <211> 12
   <212> PRT
   <213> Strongylocentrotus droebachiensis
<400> 8
   Asp Leu Arg Ala Ile Cys Ala Gly Ala His Ala Leu
   1               5                   10
<210> 9
   <211> 201
   <212> DNA
   <213> Strongylocentrotus droebachiensis
<400> 9
   ttcaaaaaga cgtttcataa agtcagtcat gcagttaaga gtggcatcca cgctggacag 60
   cgcgggtgct cggctcttgg tttttctcca gaagaaactc gcgttaaaat tctgactgcg 120
   atcccagaga tgagagaaga ggatctcagc gaagaggatc ttcgaggggc ctgcgctgct 180
   gcacatgccc ttggtcgtta g 201
<210> 10
   <211> 66
   <212> PRT
   <213> Strongylocentrotus droebachiensis
<400> 10
   Phe Lys Lys Thr Phe His Lys Val Ser His Ala Val Lys Ser Gly Ile
   1 5 10 15
   His Ala Gly Gln Arg Gly Cys Ser Ala Leu Gly Phe Ser Pro Glu Glu
   20 25 30
   Thr Arg Val Lys Ile Leu Thr Ala Ile Pro Glu Met Arg Glu Glu Asp
   35 40 45
   Leu Ser Glu Glu Asp Leu Arg Gly Ala Cys Ala Ala Ala His Ala Leu
   50 55 60
   Gly Arg
   65
<210> 11
   <211> 201
   <212> DNA
   <213> Strongylocentrotus droebachiensis
<400> 11
   ttctcaagga ccgtgcacaa cgtaggtaac gcagttagga aaggcatcca cgctggacag 60
   ggtgtatgct cgggtcttgg tctttctcca gaagaagctc gcgttaaaat tctgtctgcg 120
   gtcccagaga tgagagaaga ggatcttagc gaagaggatc ttcgagcgat ctgcgctggt 180
   gcacatgccc ttggccgtta g 201
<210> 12
   <211> 66
   <212> PRT
   <213> Strongylocentrotus droebachiensis
<400> 12
   Phe Ser Arg Thr Val His Asn Val Gly Asn Ala Val Arg Lys Gly Ile
   1               5                   10                  15
   His Ala Gly Gln Gly Val Cys Ser Gly Leu Gly Leu Ser Pro Glu Glu
               20                  25                  30
   Ala Arg Val Lys Ile Leu Ser Ala Val Pro Glu Met Arg Glu Glu Asp
           35                  40                  45
   Leu Ser Glu Glu Asp Leu Arg Ala Ile Cys Ala Gly Ala His Ala Leu
       50                  55                  60
   Gly Arg
   65
<210> 13
   <211> 48
   <212> PRT
   <213> Strongylocentrotus droebachiensis
<220>
   <221> MISC_FEATURE
   <222> (9)..(48)
   <223> Unknown amino acids are marked "Xaa" and located at position 10, 16, 25, 30, 31, 44, 45 and 48
<400> 13
   Ile Phe Gly Ser Ile Tyr His Arg Lys Xaa Val Val Lys Asn Arg Xaa
   1               5                   10                  15
   Glu Thr Val Ser Gly His Lys Thr Xaa Lys Asp Leu Thr Xaa Xaa Arg
               20                  25                  30
   Ala Val Ile Phe Arg His Glu Arg Pro Glu Val Xaa Xaa Pro Gln Xaa
           35                  40                  45
<210> 14
   <211> 49
   <212> PRT
   <213> Strongylocentrotus droebachiensis
<220>
   <221> MISC_FEATURE
   <222> (1)..(49)
   <223> unknown amino acids are marked "Xaa" and located at position 1, 1 2, 18, 27, 32, 33, 43 and 46.
<400> 14
   Xaa Asn Pro Phe Lys Lys Ile Ala Asn Arg Asn Xaa Tyr Pro Lys Thr
   1               5                   10                  15
   Thr Xaa Glu Thr Ala Gly Gly Lys Lys Thr Xaa Lys Asp Phe Ser Xaa
               20                  25                  30
   Xaa Gln Ile Val Leu Phe Gly Lys Lys Thr Xaa Ala Lys Xaa Thr Val
           35                  40                  45
   Val
<210> 15
   <211> 690
   <212> DNA
   <213> Strongylocentrotus droebachiensis
<400> 15
   atattcggaa ggatctatca tcgcaaatgc gtcgtaaaaa acagatgtga aaccgtcagc 60
   ggtcacaaga cgtgcaagga tttaacatgt tgccgagccg tgatctttag gcatgaacgc 120
   cccgaagtat gccgaccaca aacctagaca ggattcacca tggcatggga agatgaagct 180
   acccatctag agtgtattta gatcagtgac tgaatcactg aaagtctcaa accatggacc 240
   tacataggct gaagccaatg ctatactatg tcaaaccgtc gtatcttgcg tatttcattt 300
   ctctttggtt tttataatct ttcagtgttt atattctgaa atttcgaagt atccctaaaa 360
   tcaaacaact caagtttctt ttgcaaaggc attaaacaaa taaattgatc attgtttatt 420
   ttcttacata tacacaacta tacacccctt ttacgttaaa gcttccactg cctagacaga 480
   attcctaatt aagtaaagtt gaagccatcg atctagagtg tatttagatc agtagctgaa 540
   accaatgcta tactatgtca aatcgtagtt gcatgcccat ttttgttttc gttgtcataa 600
   tccttttcgt tttgaatgaa attgatatgt tttgaaatct taataaatag catggtttaa 660
   tgacaaaaaa aaaaaaaaaa aaaaaaaaaa 690
<210> 16
   <211> 660
   <212> DNA
   <213> Strongylocentrotus droebachiensis
<400> 16
   atattcggaa ggatctatca tcgcaaatgc gtcgtaaaaa acagatgcga aaccgtcaac      60
   ggtaacaaga cgtgcaagga attaacatgt tgccgagccg tgatctttag gcataaacgc     120
   cccgaagtat gccggccatc aacctagaca ggattcacca ttcatgggaa gatgaagcta     180
   cccatctaga gtgtatttag atcagtgact gaaagtctga aaccaatgct ataccatgga     240
   gatatactat gtcaaaccgt tgtatcttac gtattttatt tctttttttg gttttgataa     300
   tctttcagtg tttatatttt gacatttcga agtatcccta aaatcaaaca acttaatttt     360
   cttttgaaag gcattaaaca aataaattga tcattgttta ttttcttaca tatacaaaac     420
   tatacacccc ttttacgtta aaacttccac tgcctagaca gaattcctaa ttcagtaaag     480
   ttgaagccat cgatctagag tgtatttaga tcagtagctg aaaccaatgc tatactatgt     540
   caaatcgcag ttgcatgccg atttttgttt tcgttataat aatcctatta tcgttttgaa     600
   tgaaattgat attatattaa attcttaata aatagcatgg tttaatgaca aaaaaaaaaa     660
<210> 17
   <211> 919
   <212> DNA
   <213> Strongylocentrotus droebachiensis
<400> 17
   attccggtct gcactttagc ttgtcgttgc caaaggctgt gtcgttgagt gatctctcag      60
   tcactagctg ttcgcaagtt tccttggaag tccaaagacc agttcttatc aacccaactt     120
   caagatggat ctcaggagcg catcctttgt cttcctggtg gtagtcatgg tgctatcata     180
   ctccatggcc gcacctcttg atgcagataa cgatgaagaa atggaggaaa tcttcggctc     240
   gatctatcat cgcgaatgcg taaaaaaaaa cagatgcgaa accgtcaacg gtaacaagac     300
   gtgcaaggaa ttaacatgtt gccgagccgt gatctttagg catgaacgcc ccgaagtatg     360
   ccgaccacaa acctagacag gattcaccat ggcatgggaa gatgaagcta cccatctaga     420
   gtgtatttag atcagtgact gaatcactga aagtctcaaa ccatggacct acataggctg     480
   aagccaatgc tatactatgt caaaccgtcg tatcttgcgt atttcatttc tctttggttt     540
   ttataatctt tcagtgttta tattctgaaa tttcgaagta tccctaaaat caaacaactc     600
   aagtttcttt tgcaaaggca ttaaacaaat aaattgatca ttgtttattt tcttacatat     660
   acacaactat acaccccttt tacgttaaag cttccactgc ctagacagaa ttcctaatta     720
   agtaaagttg aagccatcga tctagagtgt atttagatca gtagctgaaa ccaatgctat     780
   actatgtcaa atcgtagttg catgcccatt tttgttttcg ttgtcataat ccttttcgtt     840
   ttgaatgaaa ttgatatgtt ttgaaatctt aataaatagc atggtttaat gacaaaaaaa     900
   aaaaaaaaaa aaaaaaaaa                                                  919
<210> 18
   <211> 558
   <212> DNA
   <213> Strongylocentrotus droebachiensis
<400> 18
   tcattttcag tgttgggttc ctcgatcatc aagatgaata ttcggaaggg atctttgatg     60
   ctcctcatca ccatgatgat cctgtctcaa gcagtggctt cacgattcct tgatgaagca    120
   gacgaagacg aacatctcat ggaggcatgg aatccgttca aaaagatagc aaatcgcaat    180
   tgttatccta aaacaacttg cgaaactgcc ggaggtaaaa aaacatgcaa ggacttcagt    240
   tgttgtcaga tcgtcctctt cggcaagaaa actcgcgcca aatgcacagt tgttacaagt    300
   tagacaccga tctggcatcc tctgacgatc ttcatgcagc agcaagtgaa ctaatgctat    360
   aacttataag acaacgaaca tcaacatacc cagagttctc ttttctcttg ataatttagt    420
   tgaacatgta agaatctaaa aatgaaaaac taagtgagca atgcaacaaa taaggatcat    480
   caattcctac taatgtatgt tgttggcaaa ataacaataa taaactgttt tgaagattga    540
   aaaaaaaaaa aaaaaaaa                                                  558
<210> 19
   <211> 556
   <212> DNA
   <213> Strongylocentrotus droebachiensis
<400> 19
   tcattttcag tgttgtgttc ctcgatcatc aagatgaata ttcgaaaggg atcattcatg     60
   ctcctcatca ccatagtgat cctgtctcaa gcagtggctt cacgattcct tgatgaagca    120
   gacgaagacg aacatctcat ggaggcatgg aatccattca aaaagatagc acatcgcaat    180
   tgttatccta aacaagagtg cgaaactgcc ggaggtaaaa aaacatgcaa ggacttcagt    240
   tgctgtcaga tcgtcctttt cggcaagaaa actcgcgcca aatgcacagt tgttacaatg    300
   agttagacac cgatctggta tcctctgacg atcttcatca gctagtgaac taatgctata    360
   acttataaga caacgaacat caacataccc aacgttctct ttttcatgat aatttagttg    420
   aacatataag aatctaaaat aaaaaattaa gtgagcaatg caacaaataa ggatcatcaa    480
   ttcctaccaa tgtatgttgt tggcaaaata aaaataatag actgtttgga aaaaaaaaaa    540
   aaaaaaaaaa aaaaaa                                                    556
<210> 20
   <211> 48
   <212> PRT
   <213> Strongylocentrotus droebachiensis
<220>
   <221> MISC_FEATURE
   <222> (1)..(48)
   <223> unknown amino acids are marked "Xaa" and located at position 45 a nd 48
<400> 20
   Ile Phe Gly Ser Ile Tyr His Arg Lys Cys Val Val Lys Asn Arg Cys
   1 5 10 15
   Glu Thr Val Ser Gly His Lys Thr Cys Lys Asp Leu Thr Cys Cys Arg
   20 25 30
   Ala Val Ile Phe Arg His Glu Arg Pro Glu Val Cys Xaa Pro Gln Xaa
   35 40 45
<210> 21
   <211> 49
   <212> PRT
   <213> Strongylocentrotus droebachiensis
<220>
   <221> MIS_FEATURE
   <222> (1)..(49)
   <223> Unknown amino acids are marked "Xaa" and located at position 1 an d 43.
<400> 21
   Xaa Asn Pro Phe Lys Lys Ile Ala Asn Arg Asn Cys Tyr Pro Lys Thr
   1 5 10 15
   Thr Cys Glu Thr Ala Gly Gly Lys Lys Thr Cys Lys Asp Phe Ser Cys
   20 25 30
   Cys Gln Ile Val Leu Phe Gly Lys Lys Thr Xaa Ala Lys Cys Thr Val
   35 40 45
   Val
<210> 22
   <211> 148
   <212> DNA
   <213> Strongylocentrotus droebachiensis
<400> 22
   atattcggga ggatctatca tcgcaaatgc gtcgtaaaaa acagatgtga aaccgtcagc 60
   ggtcacaaga cgtgcaagga tttaacatgt tgccgagccg tgatctttag gcatgaacgc 120
   cccgaagtat gccgaccaca aacctaga 148
<210> 23
   <211> 148
   <212> DNA
   <213> Strongylocentrotus droebachiensis
<400> 23
   atattcggaa ggatctatca tcgcaaatgc gtcgtaaaaa acagatgcga aaccgtcaac 60
   ggtaacaaga cgtgcaagga attaacatgt tgccgagccg tgatctttag gcataaacgc 120
   cccgaagtat gccggccatc aacctaga 148
<210> 24
   <211> 156
   <212> DNA
   <213> Strongylocentrotus droebachiensis
<400> 24
   tggaatccgt tcaaaaagat agcaaatcgc aattgttatc ctaaaacaac ttgcgaaact 60
   gccggaggta aaaaaacatg caaggacttc agttgttgtc agatcgtcct cttcggcaag 120
   aaaactcgcg ccaaatgcac agttgttaca agttag 156
<210> 25
   <211> 159
   <212> DNA
   <213> Strongylocentrotus droebachiensis
<400> 25
   tggaatccat tcaaaaagat agcacatcgc aattgttatc ctaaacaaga gtgcgaaact 60
   gccggaggta aaaaaacatg caaggacttc agttgctgtc agatcgtcct tttcggcaag 120
   aaaactcgcg ccaaatgcac agttgttaca atgagttag 159
<210> 26
   <211> 969
   <212> DNA
   <213> Strongylocentrotus droebachiensis
<400> 26
   ataaaaggga agccataatc ggtttgagtc atcaattcag gagctcatct cacccgcaac 60
   aagctgattt cagaaccttt tcaccagatt gaagctaaac tagtcaagat gatgatcaaa 120
   gtagctcttg tgctctgtgc tattgtggca accagtatgg tgtgcgccaa ggattttgaa 180
   gagcaagatg cattggacgc tttgctgaat atgatgctcc cagaggaggt tgcatctcct 240
   gatgacgctg tagccttgca aggctggttc aagaagacgt ttcataaagt cagtcatgca 300
   gttaagagtg gcatccacgc tggacagcgc gggtgctcgg ctcttggttt ttctccagaa 360
   gaagctcgcg ttaaaattct gactgcgatc ccagagatga gagaagagga tctcagcgaa 420
   gaggatcttc gaggggcctg cgctgctgca catgcccttg gtcgttagac tcggaaggga 480
   tcgtgataca acatttggaa cgcttgatgt cgattcttcc tcgagaaaca atgaaataaa 540
   agcaagaaat tatcaacttc catcatgtgt ttgcaacttc cttataatta gaatcttctt 600
   tatcactcct gtattgttaa aatccttaaa ccttgttttc cctctaaaat gaaatgatgg 660
   tgagtgtttt tcattattaa tacatatttt gcctttactt caatgtatcc aattaccata 720
   catctttcaa aggtgtttac aaatatgtag ggttaaaagt acaccaacct taaaatctag 780
   cataatacta catcttttca tcaaaagctt caaattaaaa atgttctctc tgtcttgctc 840
   ttcttttttc ctaaagaaaa gtaccaacaa caagacattg taagcatgga ataaggggaa 900
   taactaatag tattggtaat aatgctgata taaaatcaca tgaacttgca taccataaaa 960
   aaaaaaaaa 969
<210> 27
   <211> 940
   <212> DNA
   <213> Strongylocentrotus droebachiensis
<400> 27
   gatcaattca ggagctcatc tcacccgcaa caagctgatt tcagaacctt ttcaccagat 60
   tgaagctaaa ctagtcaaga tgatgatcaa agtagctctt gtgctctgtg ctattgtggc 120
   aaccagtatg gtgtgcgcca aggattttga agagcaagat gcattggacg ctttgctgaa 180
   tatgatgctc tcagaggagg ttgcatctcc tgatgacgct gtagccttgc aaggctggtt 240
   caagaagacg tttcataaag tcagtcatgc agttaagagt agcatccacg ctggacagcg 300
   cgggtgctcg gctcttggtt tttctccaga agaagctcgc gttaaaattc tgactgcgat 360
   cccagagatg agagaagagg atctcagcga agaggatctt cgaggggcct gcgctgctgc 420
   acatgccctt ggtcgttaga ctcggaaggg atcgtgatac aacatttgga acgcttgatg 480
   tcgattcttc ctcgagaaac aatgaaataa aagcaagaaa ttatcaactt ccatcatgtg 540
   tttgcaactt ccttataatt agaatcttct ttatcactcc tgtattgtta aaatccttaa 600
   accttgtttt ccctctaaaa tgaaatgatg gtgagtgttt ttcattatta atacatattt 660
   tgcctttact tcaatgtatc caattaccat acatctttca aaggtgttta caaatatgta 720
   gggttaaaag tacaccaacc ttaaaatcta gcataatact acatcttttc atcaaaagct 780
   tcaaattaaa aatgttctct ctgtcttgct cttctttttt cctaaagaaa agtaccaaca 840
   acaagacatt gtaagcatgg aataagggga ataactaata gtattggtaa taatgctgat 900
   ataaaatcac atgaacttgc ataccataaa aaaaaaaaaa 940
<210> 28
   <211> 940
   <212> DNA
   <213> Strongylocentrotus droebachiensis
<400> 28
   gatcaattca ggagctcatc tcacccgcaa caagctgcct tcagaacctt ctcaccagat 60
   tgaagctaaa ctagtcaaga tgatgatcaa agtagctctt gtgctctgtg ctattgtggc 120
   aaccagtatg gtatgcgcaa agaattttga agagcaagat gcattggaca ctttgctgaa 180
   tatgatgctc tcagaggagg ttgcatctcc tgatgacgct gtagccttgc aaggctggtt 240
   caagaagacg tttcataaag tcagtcatgc agttaagagt gacatccacg ctggacagcg 300
   cgggtgctcg gctcttggtt tttctccaga agaagctcgc gttaaaattc tgactgcgat 360
   cccagagatg agagaagagg atctcagcga agaggatctt cgaggggcct gcgctgctgc 420
   acatgccctt ggtcgttaga ctcggaaggg atcgtgatac aacatttgga acgcttgatg 480
   tcgattcttc ctcgagaaac aatgaaataa aagcaagaaa ttatcaactt ccatcatgtg 540
   tttgcaactt ccttataatt agaatcttct ttatcactcc tgtattgtta aaatccttaa 600
   accttgtttt ccctctaaaa tgaaatgatg gtgagtgttt ttcattatta atacatattt 660
   tgcctttact tcaatgtatc caattaccat acatctttca aaggtgttta caaatatgta 720
   gggttaaaag tacaccaacc ttaaaatcta gcataatact acatcttttc atcaaaagct 780
   tcaaattaaa aatgttctct ctgtcttgct cttctttttt cctaaagaaa agtaccaaca 840
   acaagacatt gtaagcatgg aataagggga ataactaata gtattggtaa taatgctgat 900
   ataaaatcac atgaacttgc ataccataaa aaaaaaaaaa 940
<210> 29
   <211> 574
   <212> DNA
   <213> Strongylocentrotus droebachiensis
<400> 29
   ataaaaggga agccataatc ggttcgagtc atcaattcag gagctcatct cacccgcaac 60
   aagctacctt aagaaccttc tcaccagatt gaagctaaac tactcaagat gatgatcaaa 120
   gtagctcttg tgctctgtgc tattgtggca accagtatgg tgtgcgcaaa gaattttgaa 180
   gagcaagatg cattggacac tttgctgaat ctaatgcttt cggaggaggc tgcatctccc 240
   gatgctgtgg ccttgcagag ctggttcagt cgcacggttc ataacgtcgg taacgcagtt 300
   aggaaaggca tccacgctgg acagggtgta tgctcgggtc ttggtctttc tccagaagaa 360
   gctcgcgtta aaattctgtc tgcggtccca gagatgagag aagaggatct tagcgaagag 420
   gatcttcgag cgatctgcgc tggtgcacat gcccttggcc gttagactcg gaagggatcg 480
   tgatacaaca tttggaacgc ttgatgtcga ttcttcctcg agaaacaatg caataaaagc 540
   aagaaattat caacttcaaa aaaaaaaaaa aaaa 574
<210> 30
   <211> 1104
   <212> DNA
   <213> Strongylocentrotus droebachiensis
<220>
   <221> Intron
   <222> (62)..(501)
   <223>
<400> 30
   atattcggaa ggatctatca tcgcaaatgc gtcgtaaaaa acagatgtga aaccgtcagc 60
   ggtaagtata tttaggctga cattatgaaa aagtattaaa cgcgtattgg aacgacttga 120
   agaaaaaaca aacagaatct ttcatttaac gttcatgaat tccgtgacta gttcaatatc 180
   atttatgtat ttattcattt gtttatttac cctaaaccta aaccttaccc caatcgcaac 240
   cctaacctat ccctatactt ttcacgtaat aaagcctgga gcagatgtcg aatcatccag 300
   aaataaagtg caagacgtac cagaaatcat aactttatga caccttttca ctgcttgaaa 360
   ttattaagaa agatattgca aagtgttttg ctatttaata atggttgtct tttaataatt 420
   cgttgttcat tgaacctgca atatcctttt ccatctcacc tggattatta tcttctttta 480
   ctttttattt tatttgtata ggtcacaaga cgtgcaagga tttaacatgt tgccgagccg 540
   tgatctttag gcatgaacgc cccgaagtat gccgaccaca aacctagaca ggattcacca 600
   tggcatggga agatgaagct acccatctag agtgtattta gatcagtgac tgaatcactg 660
   aaagtctcaa accatggacc tacataggct gaagccaatg ctatactatg tcaaaccgtc 720
   gtatcttgcg tatttcattt ctctttggtt tttataatct ttcagtgttt atattctgaa 780
   atttcgaagt atccctaaaa tcaaacaact caagtttctt ttgcaaaggc attaaacaaa 840
   taaattgatc attgtttatt ttcttacata tacacaacta tacacccctt ttacgttaaa 900
   gcttccactg cctagacaga attcctaatt aagtaaagtt gaagccatcg atctagagtg 960
   tatttagatc agtagctgaa accaatgcta tactatgtca aatcgtagtt gcatgcccat 1020
   ttttgttttc gttgtcataa tccttttcgt tttgaatgaa attgatatgt tttgaaatct 1080
   taataaatag catggtttaa tgac 1104
<210> 31
   <211> 1015
   <212> DNA
   <213> Strongylocentrotus droebachiensis
<220>
   <221> Intron
   <222> (62)..(427)
   <223>
<400> 31
   atattcggaa ggatctatca tcgcaaatgc gtcgtaaaaa acagatgcga aaccgtcaac 60
   ggtaagttta caggctgaca ttatgacaaa aagtattaaa cgtatgagtg gaacgacttg 120
   aagaaaaaaa aagaaccttt catttaacgt tcatgaattc cgcgaccagt tcattatcat 180
   ttatgtattt atgaagcctg gagcaaatgt cgaatcatcc tgaaataatg tgcaagacgt 240
   accagaaatc atcactttaa gacaccttta catgcactgc atggaaatat taagaaagat 300
   attgcaaagt gttttgctat ttaataatgg ttgtctttaa ataattcgtt tttcattgaa 360
   cctgcaatat ccttttccac ctcacctaga ttatttttgt tcttctttta cttatttatt 420
   tgtataggta acaagacgtg caaggaatta acatgttgcc gagccgtgat ctttaggcat 480
   aaacgccccg aagtatgccg gccatcaacc tagacaggat tcaccattca tgggaagatg 540
   aagctaccca tctagagtgt atttagatca gtgactgaaa gtctgaaacc aatgctatac 600
   catggagata tactatgtca aaccgttgta tcttacgtat tttatttctt tttttggttt 660
   tgataatctt tcagtgttta tattttgaca tttcgaagta tccctaaaat caaacaactt 720
   aattttcttt tgaaaggcat taaacaaata aattgatcat tgtttatttt cttacatata 780
   caaaactata cacccctttt acgttaaaac ttccactgcc tagacagaat tcctaattca 840
   gtaaagttga agccatcgat ctagagtgta tttagatcag tagctgaaac caatgctata 900
   ctatgtcaaa tcgcagttgc atgccgattt ttgttttcgt tataataatc ctattatcgt 960
   tttgaatgaa attgatatta tattaaattc ttaataaata gcatggttta atgac 1015
<210> 32
   <211> 539
   <212> DNA
   <213> Strongylocentrotus droebachiensis
<400> 32
   tcattttcag tgttgggttc ctcgatcatc aagatgaata ttcggaaggg atctttgatg 60
   ctcctcatca ccatgatgat cctgtctcaa gcagtggctt cacgattcct tgatgaagca 120
   gacgaagacg aacatctcat ggaggcatgg aatccgttca aaaagatagc aaatcgcaat 180
   tgttatccta aaacaacttg cgaaactgcc ggaggtaaaa aaacatgcaa ggacttcagt 240
   tgttgtcaga tcgtcctctt cggcaagaaa actcgcgcca aatgcacagt tgttacaagt 300
   tagacaccga tctggcatcc tctgacgatc ttcatgcagc agcaagtgaa ctaatgctat 360
   aacttataag acaacgaaca tcaacatacc cagagttctc ttttctcttg ataatttagt 420
   tgaacatgta agaatctaaa aatgaaaaac taagtgagca atgcaacaaa taaggatcat 480
   caattcctac taatgtatgt tgttggcaaa ataacaataa taaactgttt tgaagattg 539
<210> 33
   <211> 529
   <212> DNA
   <213> Strongylocentrotus droebachiensis
<400> 33
   tcattttcag tgttgtgttc ctcgatcatc aagatgaata ttcgaaaggg atcattcatg 60
   ctcctcatca ccatagtgat cctgtctcaa gcagtggctt cacgattcct tgatgaagca 120
   gacgaagacg aacatctcat ggaggcatgg aatccattca aaaagatagc acatcgcaat 180
   tgttatccta aacaagagtg cgaaactgcc ggaggtaaaa aaacatgcaa ggacttcagt    240
   tgctgtcaga tcgtcctttt cggcaagaaa actcgcgcca aatgcacagt tgttacaatg    300
   agttagacac cgatctggta tcctctgacg atcttcatca gctagtgaac taatgctata    360
   acttataaga caacgaacat caacataccc aacgttctct ttttcatgat aatttagttg    420
   aacatataag aatctaaaat aaaaaattaa gtgagcaatg caacaaataa ggatcatcaa    480
   ttcctaccaa tgtatgttgt tggcaaaata aaaataatag actgtttgg                529
<210> 34
   <211> 48
   <212> PRT
   <213> Strongylocentrotus droebachiensis
<400> 34
   Ile Phe Gly Arg Ile Tyr His Arg Lys Cys Val Val Lys Asn Arg Cys
   1               5                   10                  15
   Glu Thr Val Ser Gly His Lys Thr Cys Lys Asp Leu Thr Cys Cys Arg
               20                  25                  30
   Ala Val Ile Phe Arg His Glu Arg Pro Glu Val Cys Arg Pro Gln Thr
           35                  40                  45
<210> 35
   <211> 48
   <212> PRT
   <213> Strongylocentrotus droebachiensis
<400> 35
   Ile Phe Gly Arg Ile Tyr His Arg Lys Cys Val Val Lys Asn Arg Cys
   1               5                   10                  15
   Glu Thr Val Asn Gly Asn Lys Thr Cys Lys Glu Leu Thr Cys Cys Arg
               20                  25                  30
   Ala Val Ile Phe Arg His Lys Arg Pro Glu Val Cys Arg Pro Ser Thr
           35                  40                  45
<210> 36
   <211> 48
   <212> PRT
   <213> Strongylocentrotus droebachiensis
<400> 36
   Ile Phe Gly Ser Ile Tyr His Arg Glu Cys Val Lys Lys Asn Arg Cys
   1               5                   10                  15
   Glu Thr Val Asn Gly Asn Lys Thr Cys Lys Glu Leu Thr Cys Cys Arg
               20                  25                  30
   Ala Val Ile Phe Arg His Lys Arg Pro Glu Val Cys Arg Pro Ser Thr
           35                  40                  45
<210> 37
   <211> 51
   <212> PRT
   <213> Strongylocentrotus droebachiensis
<400> 37
   Trp Asn Pro Phe Lys Lys Ile Ala Asn Arg Asn Cys Tyr Pro Lys Thr
   1               5                   10                  15
   Thr Cys Glu Thr Ala Gly Gly Lys Lys Thr Cys Lys Asp Phe Ser Cys
               20                  25                  30
   Cys Gln Ile Val Leu Phe Gly Lys Lys Thr Arg Ala Lys Cys Thr Val
           35                  40                  45
   Val Thr Ser
       50
<210> 38
   <211> 52
   <212> PRT
   <213> Strongylocentrotus droebachiensis
<400> 38
   Trp Asn Pro Phe Lys Lys Ile Ala His Arg Asn Cys Tyr Pro Lys Gln
   1               5                   10                  15
   Glu Cys Glu Thr Ala Gly Gly Lys Lys Thr Cys Lys Asp Phe Ser Cys
               20                  25                  30
   Cys Gln Ile Val Leu Phe Gly Lys Lys Thr Arg Ala Lys Cys Thr Val
           35                  40                  45
   Val Thr Met Ser
       50
<210> 39
   <211> 945
   <212> DNA
   <213> Strongylocentrotus droebachiensis
<400> 39
   attcggcacg aggctcaccc gcaacaagct gatttcagaa ccttttcacc agattgaagc 60
   taaactagtc aagatgatga tcaaagtagc tcttgtgctc tgtgctattg tggcaaccag 120
   tatggtgtgc gccaaggatt ttgaagagca agatgcattg gacgctttgc tgaatatgat 180
   gctctcagag gaggttgcat ctcctgatga cgctgtagcc ttgcaaggct ggggcagcaa 240
   atttaagaag gcgtttcata aagtcagtca tgtagttagg caaggcatcc acgctggaca 300
   gcgtgtgtgc agcggggttg gtctttctcc agaagaagct cgcgttaaaa ttctgactgc 360
   gttcccagag atgaaagaag aggatctcag cgaagagcaa gttgcagcat actgcgctgg 420
   tgcacatgcc cttggccgtt agactcggaa gggatcgtga tacaacattt ggaacgcttg 480
   atgttgattc ttcctcgaga aaaaatgaaa tataggcaag aaattgtcaa cttccatcat 540
   gtttttgcac tttccttata tcaattagaa tcttctttat cactcctgta ttgttaaatc 600
   cttaaacctt gttttccctc taaaatgaaa tgatggtgag tggtttttat tattaataca 660
   tattttggct ttactttcat gtatccaatc attatacatc tttcaaaggt gtttacaaat 720
   atgtatggtt gaaagtacac caaccttaaa atctagcata atactacatc ttttcatcaa 780
   aagcttcaaa ttaaaaatgt tctctctgtc ttgctcttct tttttcctaa agaaaagtac 840
   caacaacaag acattgtaag catggaataa ggggaataac taatagtatt ggtaataatg 900
   ctgataataa aatcacatga acttgcaaaa aaaaaaaaaa aaaaa 945
<210> 40
   <211> 682
   <212> DNA
   <213> Strongylocentrotus droebachiensis
<400> 40
   ccgcaacaag ctgccttcag aaccttctca ccagattgaa gctaaactag tcaagatgat 60
   gatcaaagta gctcttgtgc tctgtgctat tgtggcaacc agtatggtgt gcgcaaagaa 120
   ttttgaagag caagatgcat tggacacttt gctgaatctg atgctctcag aggaggttgc 180
   atctcctgat gacgctgtgg ccttgcaagg ctggatcaag cacgcgtttc atcacgtcac 240
   tcatgcagtt agccacgcgg ctcatcaagt cagtcatgca gttaaggcag gcatccacgc      300
   tggacagggt gtgtgctcgg ggcttggtct ttctccagaa gaagctcgcg ttaaaattct      360
   gactgcgatc ccagagatga aagaagagga tctcaccgaa gagggtgttc gagcaatctg      420
   cgctggtgca catgcccttg gccgttagac tcggaaggga tcgtgataca acatttggaa      480
   cgcttgatgt cgattcttcc tcgagggggg gcccggtacc caattcgccc tatagtgagt      540
   cgtattacaa ttcactggcc gtcgttttac aacgtcgtga ctgggaaaac cctggcgtta      600
   cccaacttaa tcgccttgca gcacatcccc ctttcgccag ctggcgtaat agcgaagagg      660
   cccgcaccga tcgcccttcc ca                                               682
<210> 41
   <211> 20
   <212> PRT
   <213> Artificial
<400> 41
   Gly Trp Phe Lys Lys Thr Phe His Lys Val Ser His Ala Val Lys Ser
   1               5                   10                  15
   Gly Ile His Ala
               20
<210> 42
   <211> 10
   <212> PRT
   <213> artifical
<400> 42
   Gly Trp Phe Lys Lys Thr Phe His Lys Val
   1               5                   10
<210> 43
   <211> 5
   <212> PRT
   <213> artifical
<400> 43
   Gly Trp Phe Lys Lys
   1               5
<210> 44
   <211> 10
   <212> PRT
   <213> artifical
<400> 44
   Ser His Ala Val Lys Ser Gly Ile His Ala
   1               5                   10
<210> 45
   <211> 10
   <212> PRT
   <213> artifical
<400> 45
   Gly Gln Arg Gly Cys Ser Ala Leu Gly Phe
   1               5                   10

## Description

### FIELD OF THE INVENTION

The present invention relates to novel polypeptides, nucleic acids encoding said peptides, cell lines, vectors, pharmaceutical compositions and antibodies. The invention may be used to treat fungus, infections, ulcers, wounds, cancer and/or inflammation in a patient in need thereof, as well as to be used as indicators to detect e.g. animal welfare.

### BACKGROUND OF THE INVENTION

### Antimicrobial peptides (AMPs)

The evolution of antibiotic-resistant pathogenic bacteria has stimulated the search for alternative antimicrobial agents from alternative sources like the marine environment (De Vries and Hall, 1994). Echinoderms (sea urchins, sea cucumbers, sea stars, sea lilies) are benthic organisms, which are constantly exposed to relatively high concentrations of bacteria, viruses and fungi of which many may be harmful to the organism. The survival of these organisms depends on efficient antimicrobial mechanisms to protect themselves against microbial infections and fouling. Rinehart *et al.* (1981) examined 83 unidentified species of echinoderms from the west coast of Baja California and the Gulf of California and found 43% to have antimicrobial activity. In the same study, 58% out of 36 unidentified Caribbean species showed antimicrobial activity. Out of 22 species of echinoderms collected from the northern Gulf of Mexico, 80% had antimicrobial activity (Bryan *et al.,* 1994). These results support that marine echinoderms are a potential source for new types of antibiotics for pharmaceutical use.

A variety of Antimicrobial compounds, including steroidal glycosides (Andersson *et al.*, 1989), polyhydroxylated sterols (Iorizzi *et al.,* 1995), naphthoquinone pigments (Service and Wardlaw, 1984), lysozymes (Canicatti and Roch, 1989; Stabili and Pagliara, 1994), and complement-like substances (Leonard *et al.,* 1990) have been isolated from echinoderms. Results from recent publications (Beauregard *et al.,* 2001; Haug et al., 2002b) indicate the presence of antimicrobial peptides/proteins in echinoderms, including sea urchins.

Gene-encoded, ribosomally synthesized antimicrobial peptides (AMP) are widespread in nature, and seem to be important defence molecules in different phyla of the plant and animal kingdom. Many antimicrobial peptides show a high specificity for prokaryotes and a low toxicity for eukaryotic cells, and their mode of action (Hancock and Rozek, 2002) is considered less likely to lead to development of resistance. These properties have favoured their investigation and exploitation as potential new antibiotics (Bax *et al.*, 2000; Mor, 2000). Most naturally occurring AMPs carry a net positive charge (i.e. cationic) and are composed of 12-50 residues where more than 50% of them are hydrophobic (Hancock and Diamond, 2000). In membrane-like (non-polar) environments they tend to form amphipathic structures, i.e. structures with separate hydrophobic and hydrophilic domains. Their positive charge presumably facilitates interactions with the negatively charged bacterial phospholipid-containing membranes and/or acidic bacterial cell walls, whereas their amphipathic character enables membrane permeabilization (Devine and Hancock, 2002).

Based on their amino acid sequences and secondary structures, AMPs have been classified into four major groups (Devine and Hancock, 2002): (a) Linear, α-helical peptides without cysteine, (b) Linear peptides with an extended structure, characterized by an unusual proportion of one or more amino acids, most often proline, tryptophan, histidine or glycine, (c) Peptides with one disulphide bond, giving a looped structure, and (d) Peptides with two ore more disulphide bonds, with mainly or exclusively β-sheet structure. An additional group may be added for the θ-defensins, which are circular peptides made from two genes with a novel biosynthesis (Tang *et al.*, 1999). Most cationic peptides selectively interact with anionic bacterial membranes, although different killing mechanisms may be used by different peptides (Epand and Vogel, 1999).

Invertebrates are proving to be a rich source of AMPs, possibly because of their more pronounced reliance on innate immune functions in their defence against microbial pathogens than vertebrates. Although the marine environment comprises approximately half of the total global biodiversity (De Vries and Hall, 1994), less than 5% of the AMPs deposited in the Antimicrobial Sequences Database (http://www.bbcm.univ.trieste.it/~tossi/pag1.htm) are of marine origin. Many of the peptides and proteins isolated from marine species are structurally unique and display significant biological activities. Marine peptides are therefore expected to be useful drug leads or potential drugs.

Antimicrobial peptides containing brominated tryptophan have earlier been disclosed. E.g., Hagfish intestinal antimicrobial peptides (HFIAP's), isolated from Atlantic hagfish, *Myxine glutinosa* and Styelin D, an antimicrobial peptide isolated from a tunicate, *Styela clavata*. In addition, carnivorous marine cone snails (*Conus* spp.) produce potent neurotoxic peptides containing brominated tryptophan residues. Morulin Pm, a polypeptide containing 6-Bromotryptophan was isolated from the morula cells of the ascidian, *Phallusia mammillata*. The unusual amino acid bromotryptophan may make the active peptides less susceptible to proteolysis for steric reasons. Such protease resistance could extend the pharmacokinetic lifetimes of the peptides *in vivo*, sustaining antimicrobial activity. The post-translational modification may also increase the biological activity of the peptides.

Previously it has been reported detection of antibacterial activity against several bacterial strains in coelomocyte extracts of the green sea urchin *Strongylocentrotus droebachiensis* (Haug et al., 2002b). Sensitivity to protease treatment indicated that at least some of the active components were of protein nature. However, no further characterization was provided.

The present invention discloses a distinct and unique group of polypeptides. These novel polypeptides are isolated from sea urchin, more particularly from the blood cells of the green urchin, *Strongylocentrotus droebachiensis.* All peptides of the invention are able to kill or inhibit growth of bacteria like *Escherichia coli, Staphylococcus aureus, Listionella anguillarum and Corynebacterium glutamicum.* Most interestingly, the peptides of the present invention are the first polypeptides from sea urchins with antimicrobial activity that have been purified and characterised.

### SUMMARY OF THE INVENTION

The present invention discloses a distinct and unique group of polypeptides. These novel polypeptides are isolated from sea urchin, more particularly from the blood cells of the green urchin, *Strongylocentrotus droebachiensis*. Most interestingly, the peptides of the present invention are the first polypeptides from sea urchins with antimicrobial activity that have been purified and characterised. The present invention also relates to DNA or RNA encoding said peptides, pharmaceutical preparations comprising said peptides, antibodies that specifically bind with any one of said peptides, vectors, cell lines as well as to methods for the production of said peptides and to different uses of said peptides, nucleic acids and vectors. In addition to antibacterial effects, the peptides of the present invention may be effective against ulcers, fungi, parasites, viral infections, inflammation, wounds as well as cancer.

### DESCRIPTION OF THE FIGURES

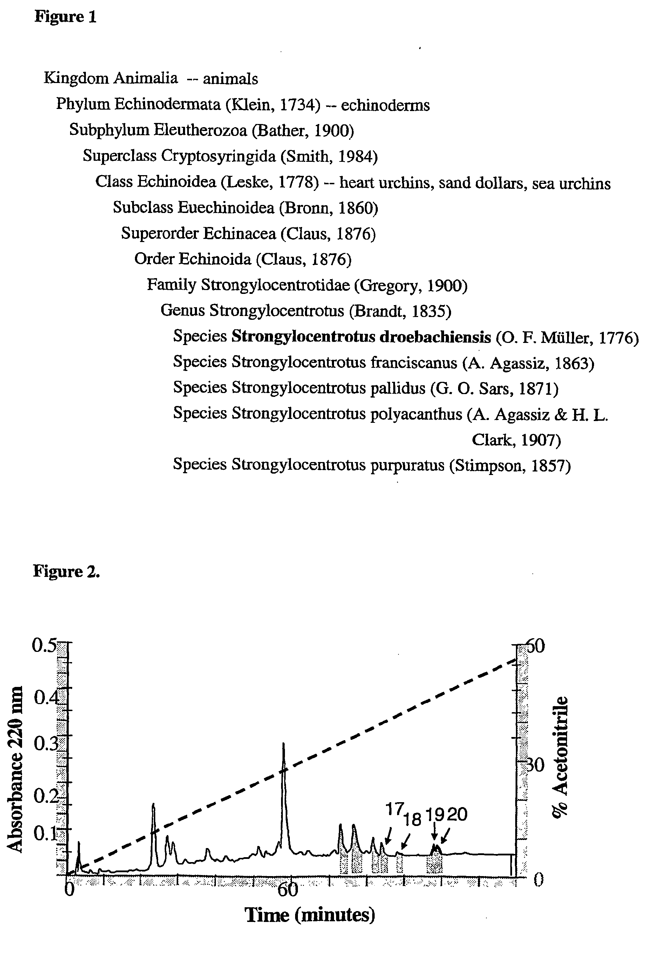

**Figure 1****.** Taxonomic hierarchy of the Genus *Strohgylocentrotus*.
**Figure 2****.** Purification of antibacterial peptides from *S. droebachiensis* coelomocytes by reverse-phase high performance liquid chromatography (RP-HPLC). A coelomocyte extract was pre-purified by solid phase extraction using Sep-Pak C₁₈ cartridges. The fraction eluted with 40% acetonitrile was subjected to RP-HPLC using a semi-preparative C₈ column. Elution was performed with a linear gradient of 0-60% acetonitrile for 120 min at a flow rate of 2 ml/min. Fractions with growth inhibiting activity against *Corynebacterium glutamicum* and *Listonella anguillarum* (numbered peaks) were submitted to further purification on an analytical C₁₈ column. The solid line shows the absorbance at 220 nm, whereas the dashed line shows the concentration of acetonitrile in the eluting solvent.

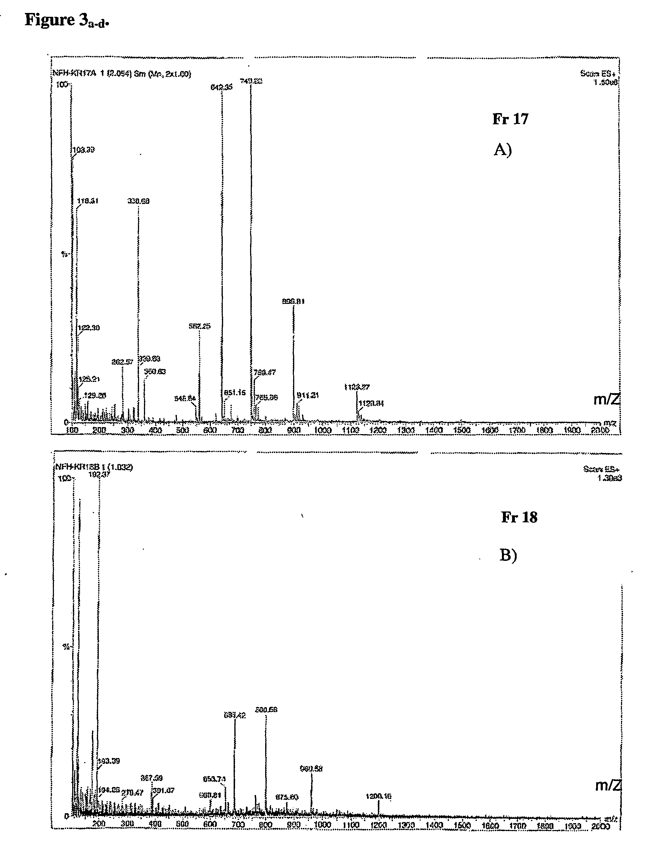

**Figure 3a****.** Electrospray ionization (ESI Q-TOF) mass spectra of the purified peptide, P17.
**Figure 3b****.** Electrospray ionization (ESI Q-TOF) mass spectra of the purified peptide, P18.

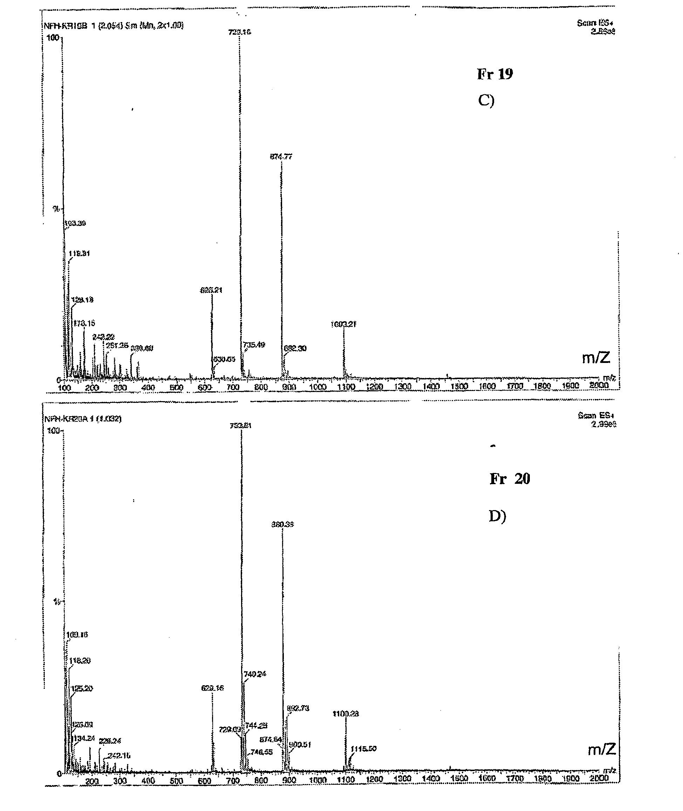

**Figure 3c****.** Electrospray ionization (ESI Q-TOF) mass spectra of the purified peptide, P19.
**Figure 3d****.** Electrospray ionization (ESI Q-TOF) mass spectra of the purified peptide, P20.

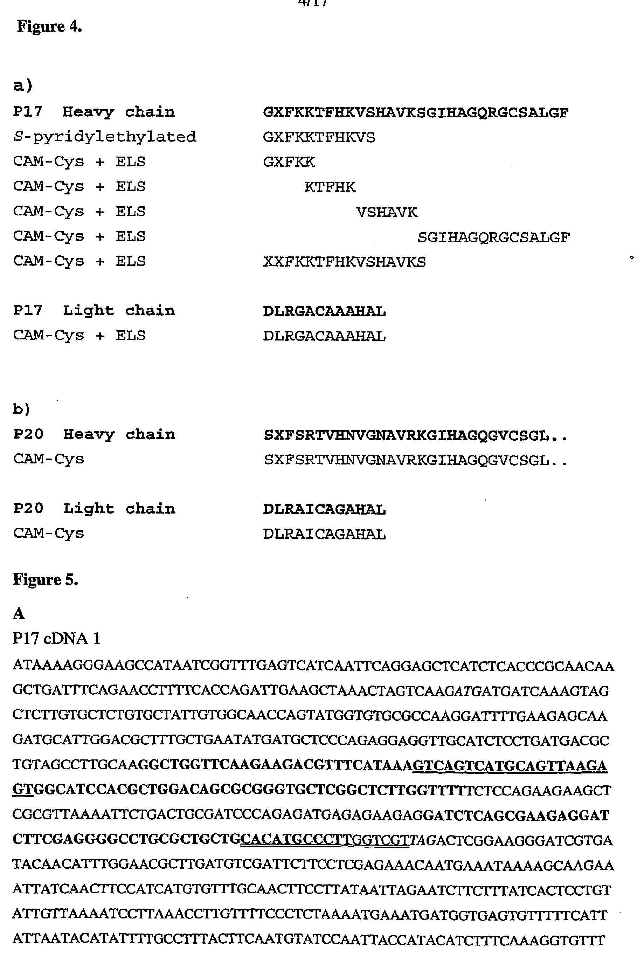

**Figure 4****.** Edman degradation sequencing of P17 (a) and P20 (b) after alkylation and proteinase treatment. N-terminal sequences were obtained after reduction and alkylation using 4-vinylpyridine (*S*-pyridylethylated) or iodoacetamide (CAM-Cys), followed by separation of the heavy and light chain by HPLC and Edman degradation. Peptide fragment sequences were obtained after Endoproteinase Lys-C (ELC) digestion, followed by separation of fragments by HPLC and Edman degradation. X denotes unknown residues. Bold one letter aminoacid symbols represents P 17 heavy chain (SEQIDNO1), P17 light chain (SEQIDNO2), P20 heavy chain (SEQIDNO7) and P20 light chain (SEQIDNO8) respectively.

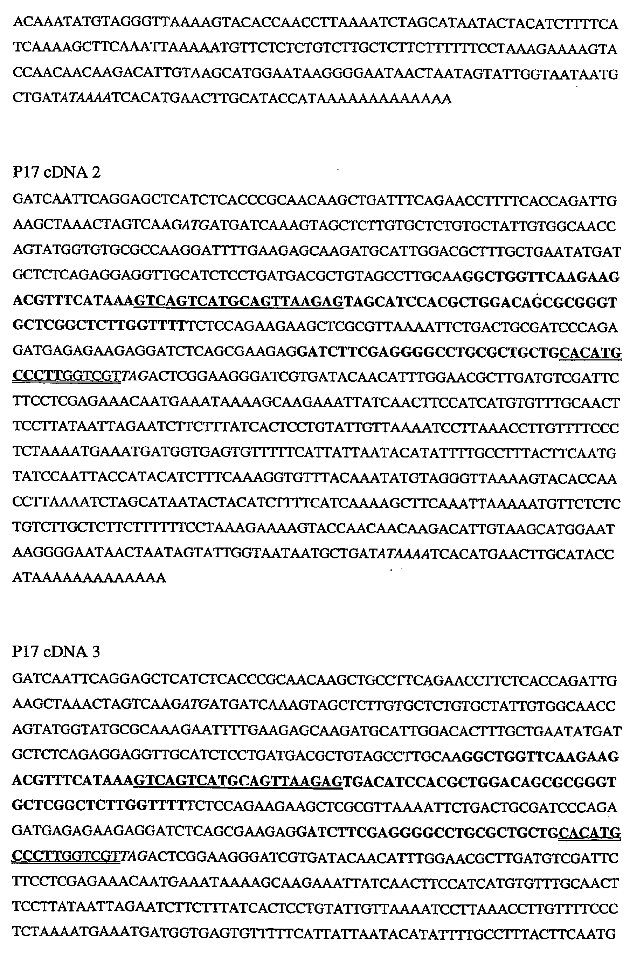

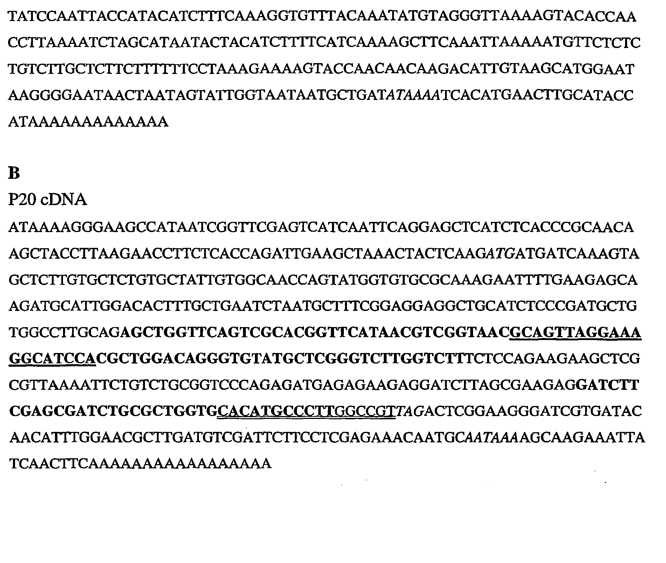

**Figure 5****.** Complete nucleotide sequences (cDNA) corresponding to the peptides P17 (A) (cDNA1 (SEQIDNO26), cDNA2 (SEQIDNO27), cDNA3 (SEQIDNO28)) and P20 (B) (cDNA (SEQIDNO29)). Nucleotide sequences annotated to show the sense (single underline) and antisense primers (double-underline). The coding sequences of mature peptides are marked in bold letters. The translation start codon (atg), stop codon (tag) and polyadenylation signal sequence (aataaa) are marked in italic, respectively.

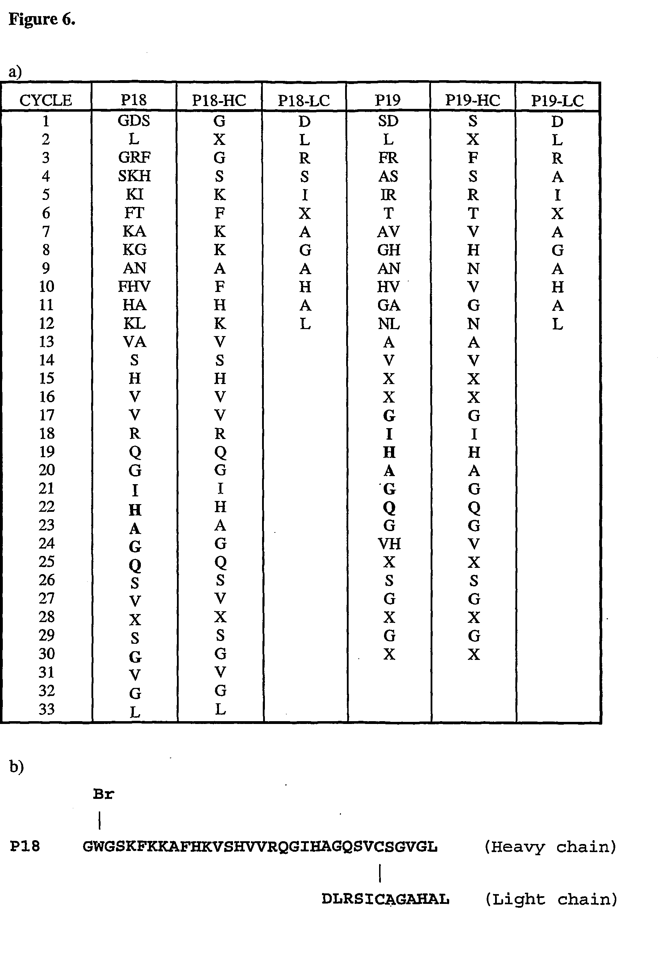

**Figure 6****.** Edman degradation and deduced amino acid sequences of P 18 and P 19 and proposed structure of P18. (a) The heavy chain (HC) of P18 and P19 were deduced by subtracting the light chain (LC), which in turn was deduced by its probable similarity to the light chain of P20. (b) The proposed structure of P18 is based on its sequence similarity to P20 (LC) and P 17 (HC) and by comparing calculated and measured mass of the peptide. X denotes unknown residues.

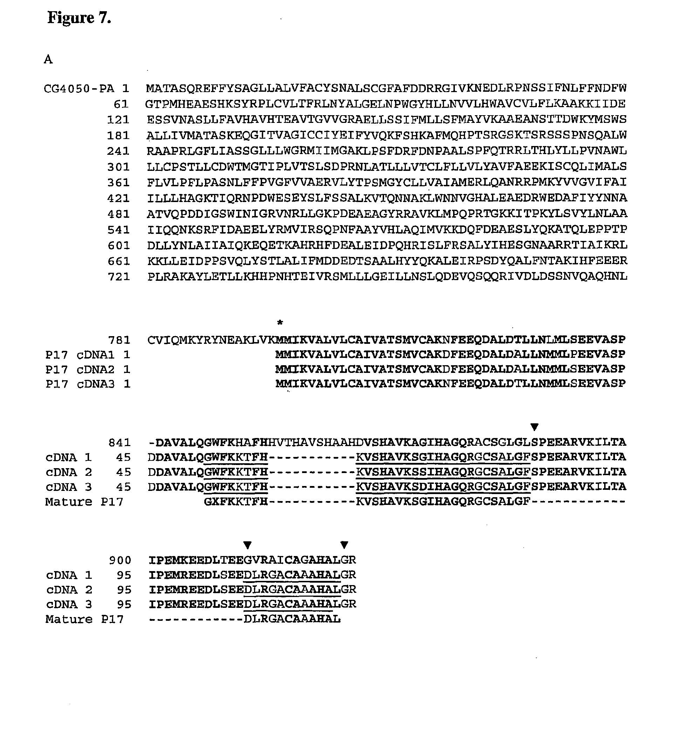

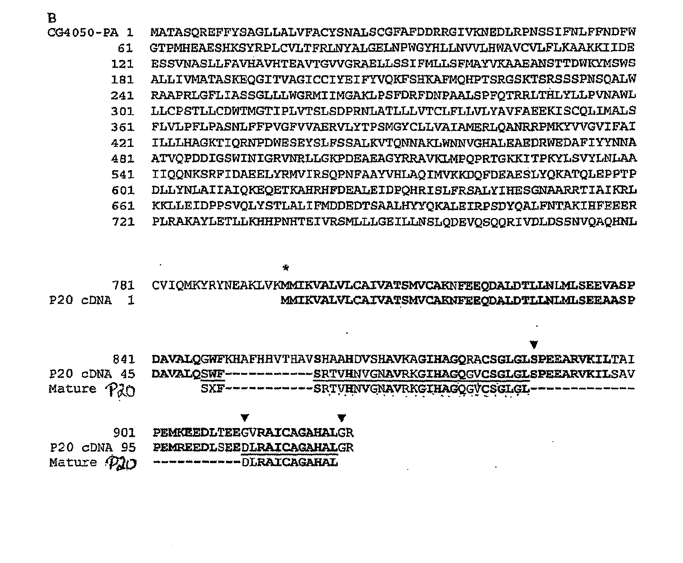

**Figure 7****.** Alignment of the deduced amino acids sequences corresponding to the peptides P17 (A) and P20 (B) isolated from the cDNA of *S. droebachiencis* and genes of the genome (whole genome shotgun sequences CG-4050 gi|721589111|ref|XM_783870.1|) of *S. purpuratus*. The identical amino acid residues are indicated by bold. The predicted cleavage site is shown by solid triangle (▼). The first amino acid residue in predicted peptide is labelled by asterisk (*). The amino acids corresponding to the proposed mature peptide in *S. strongylocentrotus* is underlined.

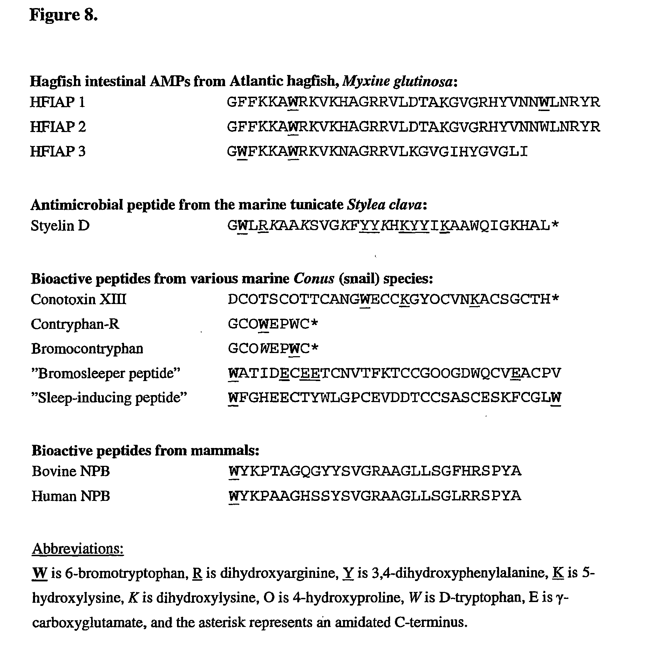

**Figure 8****.** Amino acid sequences of other selected bromotryptophan-containing bioactive peptides mentioned in the text.

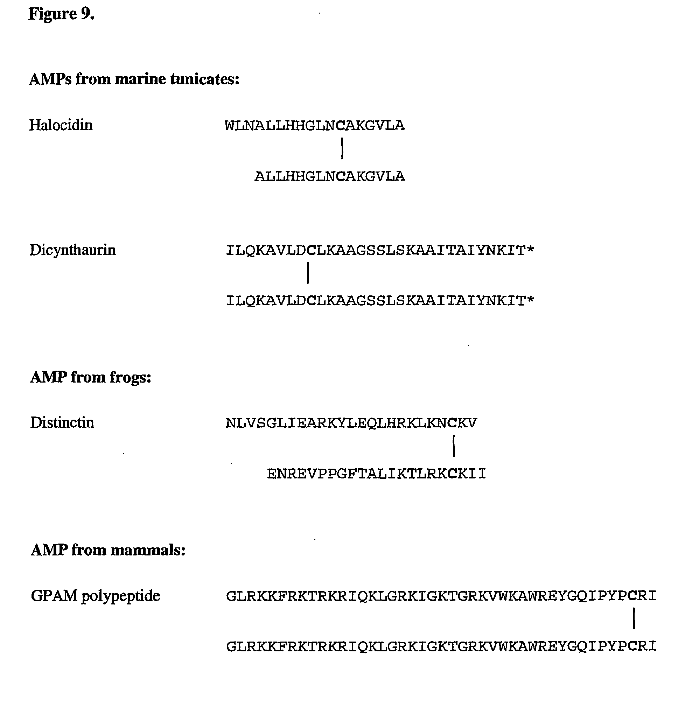

**Figure 9****.** Amino acid sequences of other dimeric antimicrobial peptides mentioned in the text.

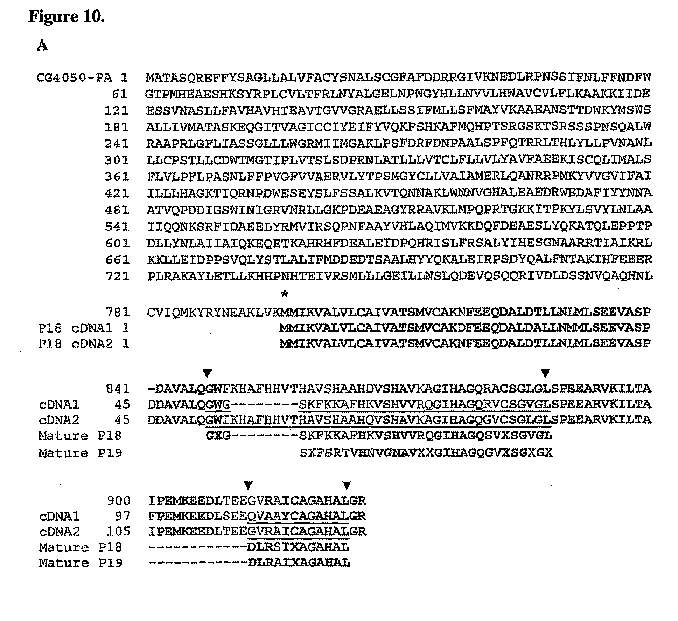

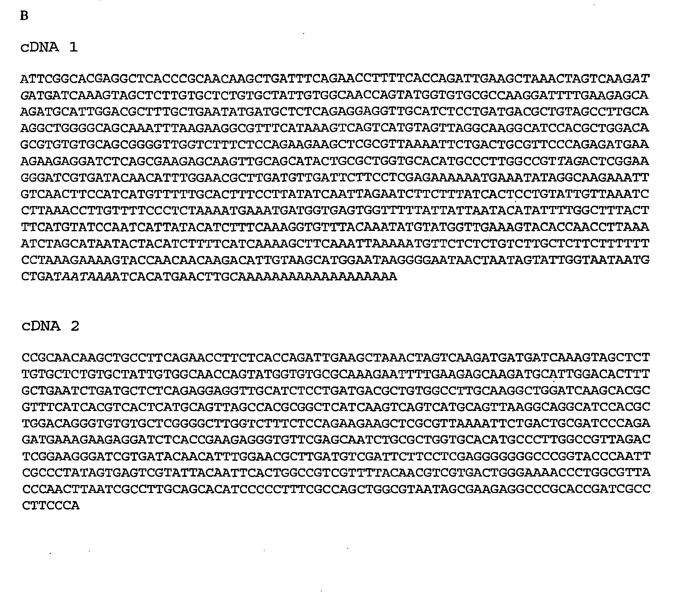

**Figure 10****.** A. Alignment of the deduced amino acids sequences from cDNA 1 and cDNA 2 (having similarity to the mature peptides P18 and P19), isolated from the cDNA library of *S. droebachiensis*, with the isolated peptides P18 and P 19, and a gene from the genome (whole genome shotgun sequences CG-4050 gi|72158911|ref|XM_783870.1|) of *S. purpuratus*. The identical amino acid residues are indicated by bold. The predicted cleavage site is shown by solid triangle (▼). The first amino acid residue in the predicted peptide is labelled by asterisk (*). The amino acids corresponding to the proposed mature peptide in *S. droebachiensis* is underlined. The complete cDNA sequences of the two cDNA clones are presented (B) (cDNA 1 (SEQIDNO39), cDNA 2 (SEQIDNO40)).

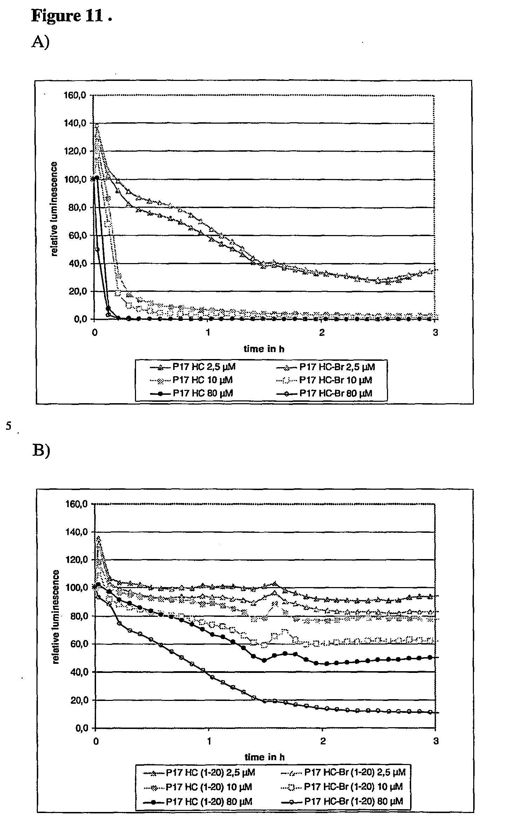

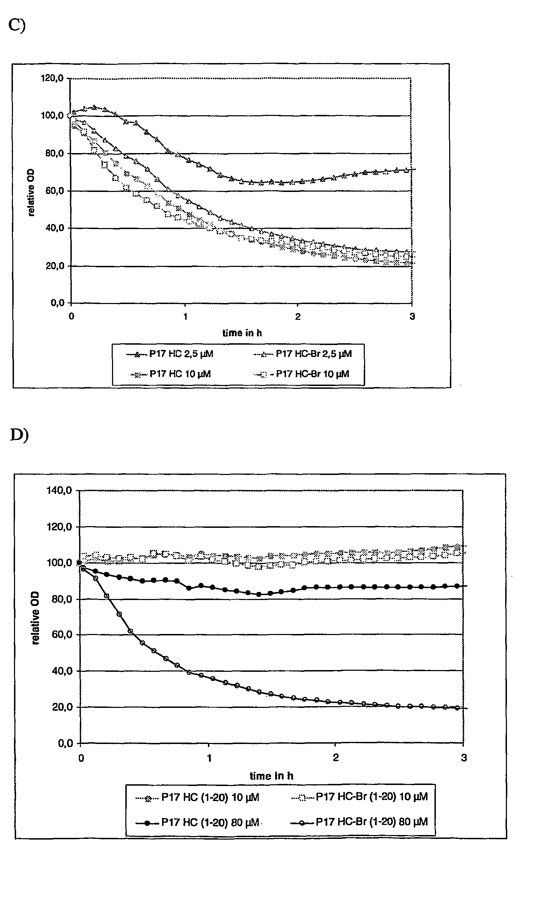

**Figure 11****.** Luminescence (A and B) and OD₆₀₀ (C and D) were followed in *Salmonella enterica* after the addition of P17 HC or P17 HC-Br (A and C), and P17 HC (1-20) or P17 HC-Br (1-20) (B and D). Measurements in absence of peptides were used as the reference. All values are plotted relative to the reference value of the respective time point which is set to 100. Growth and luminescence were followed over a period of 3 hours in a 5 minute measurement-interval. Representative data from a total of 3 measurements is shown.

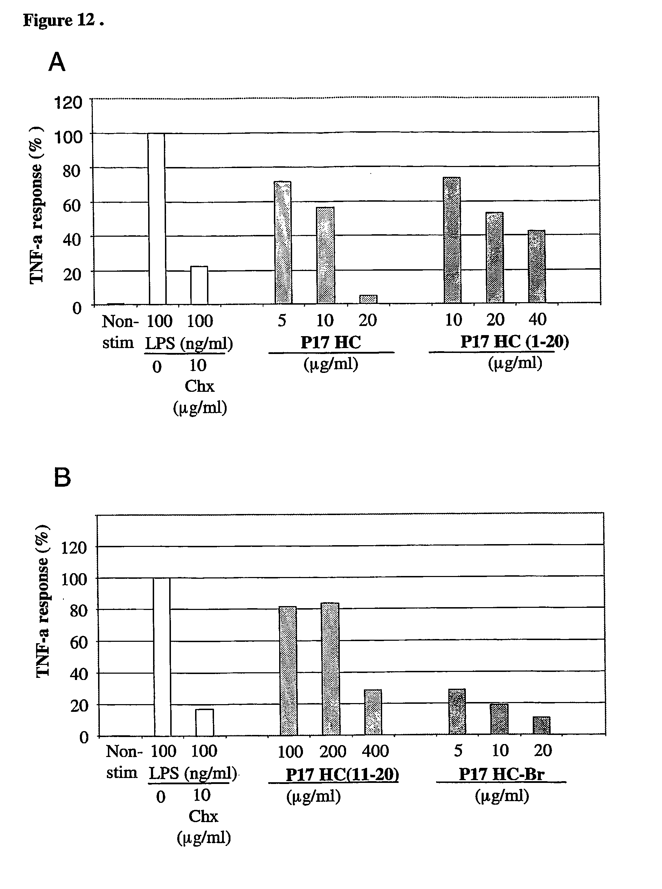

**Figure 12****.** Antiinnflammatory response measured as inhibition of tumor necrosis factor alpha (TNF-α) response in THP-1 cells after stimulation with LPS. THP-1 cells (1 x 10⁶ cells/ml) were treated with P 17 derivates in different concentrations for 1 hour, and subsequently stimulated with 100 ng/ml LPS for 6 hours. LPS was used as control for induction of TNF-α. (set as 100 % response), RPMI media was used as negative (non-stimulated) control, and cycloheximide (Chx, 10 µg/ml) was used as a an inhibitory control for LPS induced TNF-α. The data presented are the average of two parallells. (A) The effect of the peptides P17 HC and P 17 HC (1-20). (B) The effect of the peptides P17 HC (11-20) and P17 HC-Br.

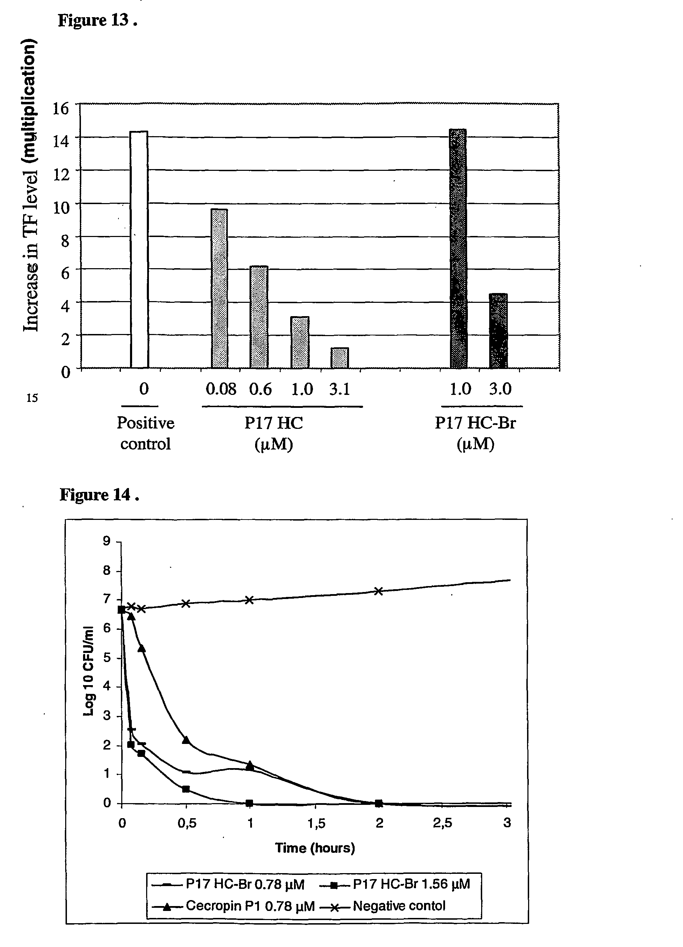

**Figure 13****.** The effect of P17 HC and P17 HC-Br on tissue factor levels (x times increase compared to negative control) in mononuclear cells after stimulation with LPS. NaCl (0.15M) was used as negative control, whereas LPS alone was used as positive control.
**Figure 14****.** Killing of *E. coli* by P17 HC-Br at peptide concentrations of 0.78 and 1.56 µM. The killing assay was done in MHB at 22°C, and bacteria added media or cecropin P1 (0.78 µM) were used as negative and positive control, respectively. The data represent the mean of two duplicates. After 5 minutes of treatment with 0.78 µM P17 HC-Br, colony forming units were reduced by approximately 10⁴, and after 2 hours, no cfu were observed.

### DETAILED DESCRIPTION OF THE INVENTION

A first aspect of the present invention relates to a polypeptide comprising one of the following amino acid sequences:
a) amino acid sequence SEQIDNO41;
b) amino acid residues 1-20 of SEQIDN03;
c) amino acid residues 1-20 of SEQIDNO5;
d) amino acid residues 1-20 of SEQIDNO7;
wherein amino acid residue number 2 is tryptophan or brominated tryptophan;
said polypeptide having antimicrobial activity.

In a first embodiment according to the first aspect of the present invention, said polypeptide comprises one of the following amino acid sequences:
a) SEQIDNO1;
b) SEQIDNO3;
c) SEQIDNO5;
d) SEQIDNO7.

In a second embodiment according to the first aspect of the present invention, said polypeptide is selected from the group consisting of:
a) SEQIDNO1;
b) SEQIDNO3;
c) SEQIDNO5; and
d) SEQIDNO7.

In a preferred embodiment of the present invention, the above mentioned polypeptides constitute a first monomer of a dimeric polypeptide. The second monomer of the dimeric peptide may comprise the amino acid sequence DLRxxxAxAHAL; x being any amino acid. Furhermore, the latter motif may be comprised in one of the following amino acid sequences;
DLRGACAAAHAL (SEQIDNO 2) (Fig. 4a, Light chain) or
DLRSIXAGAHAL (SEQIDNO 4) or
DLRAIXAGAHAL (SEQIDNO 6) or
DLRAICAGAHAL (SEQIDNO 8) (Fig4b, Light chain);
X being any amino acid residue.

In a preferred embodiment of the invention, the mentioned dimeric polypeptide comprises a first monomer with the amino acid sequence SEQIDNO1 and a second monomer with the amino acid sequence SEQIDNO2, herein referred to as P17.

The dimeric structure is preferably formed via a disulfide linkage between cysteines of two different sized monomers, more preferably between Cys25 in SEQIDNO1 and Cys6 in SEQIDNO2. Said polypeptide optionally contains a modified amino acid in position 2 of SEQIDNO1.

The cDNA coding for the partial sequence of P17 is:
...TTCAAAAAGACGTTTCATAAAGTCAGTCATGCAGTTAAGAGTGGCATCCA CGCTGGACAGCGCGGGTGCTCGGCTCTTGGTTTTTCTCCAGAAGAAACTCGC GTTAAAATTCTGACTGCGATCCCAGAGATGAGAGAAGAGGATCTCAGCGAA GAGGATCTTCGAGGGGCCTGCGCTGCTGCACATGCCCTTGGTCGTTAG (SEQIDNO 9), and the partial sequence deduced from said cDNA is:
...FKKTFHKVSHAVKSGIHAGQRGCSALGFSPEETRVKILTAIPEMREEDLSEEDL RGACAAAHALGR (SEQIDNO10).
The underlined parts in the sequence deduced from cDNA are cleaved off in the active, purified peptide.

Blast (NCBI) search of the partial cDNA sequence revealed relatively high similarity to a sequence that was deduced from mRNA of *Strongylocentrotus purpuratus*, a closely related specie to *S. droebachiensis.* The aligned sequence is the C-terminal part of a larger protein, designed "similar to CG4050-PA, isoform A (LOC583985)".

Said Blast (NCBI) search of the partial cDNA sequence also revealed similarity to a sequence deduced from RNA of *Stylea clava*, an ascidian (tunicate). The aligned sequence is a part of Styelin, an antimicrobial peptide isolated from this specie.

Furthermore, BLAST search against genomic databases of the purple sea urchin, *S. purpuratus*, show high similarity to several genome sequences. The functions of the peptides/proteins that these genes codes for are hitherto unknown.

In a preferred embodiment of the invention, the mentioned dimeric polypeptide comprises a first monomer with the amino acid sequence SEQIDNO41 and a second monomer with the amino acid sequence SEQIDNO2, herein referred to as P17(1-20). Said polypeptide optionally contains a modified amino acid in position 2 of SEQIDNO41.

In a preferred embodiment of the invention, the dimeric polypeptide of the invention comprises a first monomer with the amino acid sequence SEQIDNO7 and a second monomer with the amino acid sequence SEQIDNO8, herein referred to as P20. In another embodiment according to the present invention, said first monomer comprises amino acid residues 1-20 of SEQIDNO7.

The dimeric structure is preferably formed via a disulfide linkage between cysteines of two different sized monomers, more preferably between Cys25 in SEQIDNO7 and Cys6 in SEQIDNO8 Said polypeptide optionally contains a modified amino acid in position 2 of SEQIDNO7.

The cDNA coding for the partial sequence of P20 is:
...TTCTCAAGGACCGTGCACAACGTAGGTAACGCAGTTAGGAAAGGCATCCA CGCTGGACAGGGTGTATGCTCGGGTCTTGGTCTTTCTCCAGAAGAAGCTCGC GTTAAAATTCTGTCTGCGGTCCCAGAGATGAGAGAAGAGGATCTTAGCGAA GAGGATCTTCGAGCGATCTGCGCTGGTGCACATGCCCTTGGCCGTTAG (SEQIDNO 11), and the partial sequence deduced from said cDNA is:
...FSRTVHNVGNAVRKGIHAGQGVCSGLGLSPEEARVKILSAVPEMREEDLSEE DLRAICAGAHALGR (SEQIDNO 12).
The underlined parts in the sequence deduced from cDNA are cleaved off in the active, purified peptide.

BLAST search against genomic databases of the purple sea urchin, *S. purpuratus*, show high similarity to several genome sequences. The functions of the peptides/proteins that these genes codes for are hitherto unknown.

In a preferred embodiment of the invention, the dimeric polypeptide of the invention comprises a first monomer with the amino acid sequence SEQIDNO3 and a second monomer with the amino acid sequence SEQIDNO4, herein referred to as P 18. In another embodiment according to the present invention, said first monomer comprises amino acid residues 1-20 of SEQIDNO3.

The dimeric structure is preferably formed via a disulfide linkage between cysteines of two different sized monomers, more preferably between Cys28 in SEQIDNO3 (X28=C) and Cys6 in SEQIDNO4 (X6=C). Said polypeptide optionally contains a brominated tryptophan in position 2 of SEQIDNO3 (X= brominated tryptophan).

In a preferred embodiment of the invention, the dimeric polypeptide of the invention comprises a first monomer with the amino acid sequence SEQIDNO5 and a second monomer with the amino acid sequence SEQIDNO6, herein referred to as P19. In another embodiment according to the present invention, said first monomer comprises amino acid residues 1-20 of SEQIDNO5.

The dimeric structure is preferably formed via a disulfide linkage between cysteines of two different sized monomers, more preferably between Cys25 in SEQIDNO5 (X25=C) and Cys6 in SEQIDNO6 (X6=C). Said polypeptide optionally contains a modified amino acid in position 2 of SEQIDNO5. Preferably X25 in SEQIDNO5 and X6 in SEQIDNO6 are cysteines.

In a preferred embodiment of the current invention the amino acid residue in position 2 of SEQIDNO1, SEQIDNO41, SEQIDNO42, SEQIDNO43, SEQIDNO3, SEQIDNO5 or SEQIDNO7 may be a tryptophan or brominated tryptophan, like e.g. 5- or 6-bromotryptophan. The above polypeptides may be a monomer or a dimer, however said polypeptides are preferably dimers, or in a dimeric form. Said dimeric polypeptides have preferably a molecular mass in the range 3.5 to 5.5 kDa, more preferably in the range 4.0 to 5.0 kDa, and even more preferably in the range 4.3 to 4.8 kDa (see Table 6). The top and bottom values are included in the range.

Heterodimeric, antimicrobial peptides have previously been isolated from tunicates (Halocidin) and frogs (Distinctin). The sea urchin peptides show no homology to these peptides, indicating that they belong to a novel peptide class that has antimicrobial activity.

The polypeptides of the current invention are preferably derived from marine organisms, more preferably from *Echinoidea*, e.g. heart urchins, sand dollars, sea urchins, etc, more particularly the polypeptides are derived from animals of the subclass *Euechinoidea*, or the superorder *Echinacea* or the order *Echinoida* or the family *Strongylocentrotidae* or more particularly from the genus *Strongylocentrotus* or even more particularly from the species *Strongylocentrotus droebachiensis.*

Another aspect of the present invention relates to a nucleic acid encoding the polypeptides described above. The nucleic acid can be a DNA or a RNA. The nucleic acid sequence can be deduced by the skilled artisan on the basis of the disclosed amino acid sequences.

Furthermore, the invention relates to vectors comprising the above nucleic acids. The vectors can be of any type suitable e.g. for expression of said polypeptides or propagation of genes encoding said polypeptides in a particular organism. The organism may be of eukaryotic or prokaryotic origin. The specific choice of vector depends on the host organism and is known to a person skilled in the art.

Yet another aspect of the invention is a suitable host cell comprising said nucleic acid or vector. The suitable host cell may be any cell of eukaryotic or prokaryotic origin that is suitable e.g. for expression of said polypeptides or propagation of genes encoding said polypeptides. The eukaryotic cell line may be of mammalian, of yeast, invertebrate or sea urchin origin. The specific choice of cell line is known to a person skilled in the art. Choice of bacterial strain will depend on the task at hand and is known to a person skilled in the art.

The invention also relates to a method of producing the peptides of the invention comprising cultivating the host cell of the invention, collecting peptides expressed from the cell population; and purifying the expressed peptide.

The invention also relates to polypeptides prepared according to said method.

Another aspect of the present invention relates to the polypeptides, the nucleic acid, the antobodies or the vector of the invention for medical use.

Furthermore, the invention relates to the use of the polypeptides, the nucleic acid or the vector according to the invention for manufacturing a medicament for the treatment of infections, wounds, ulcers, cancer and/or inflammation in a patient in need thereof. Said infections may be e.g. bacterial infection and/or fungal infections and/or viral infections. The polypeptides, the nucleic acid or the vector of the current invention may also be used to stimulate the immune system.

The invention also relates to precursors of the peptides, according to the present invention, extended at the N-terminal and to recombinant materials encoding said precursors.

Furthermore, the present invention also relates to compositions and pharmaceutical compositions comprising the polypeptides, the antibodies, the DNA or RNA and/or vectors according to the invention. Said pharmaceutical compositions may also comprise a suitable pharmaceutical vehicle.

The present invention further relates to antibodies or antibody fragments that specifically binds with the polypeptide according to the second embodiment of the first aspect of the present invention.

### MATERIALS AND METHODS

### Experimental Animals and Sample Collection

Live specimen of the green sea urchin *Strongylocentrotus droebachiensis* (45 specimens) were obtained by trawling off the coast of Tromsø, Norway. All animals were collected in the period from March until May 2000, and the animals were kept in separate tanks with circulating seawater until bleeding and tissue collection. Coelomic fluid (CF) was withdrawn from the coelom by puncture of the body wall. Coelomic fluid (1260 ml totally) was immediately centrifuged at 800 g at 4° C for 20 minutes to separate the coelomocytes from the plasma (cell-free coelomic fluid). The coelomocytes were pooled, freeze-dried and kept frozen at -20° C until extraction.

The CF for the RNA collection was mixed with an equal volume of ice-cold calcium and magnesium free sea water containing 70 mM EDTA and 50 mM imidazole according to Multerer and Smith (2004). All samples were centrifuged by 6,500g for 5 min at 4°C and the supernatant was discarded. The pellets of coelomocytes were stored in -80°C freezer until further use.

### Bacterial Strains and Growth Conditions

The Gram-negative bacteria *Listonella (Vibrio) anguillarum*, serotype 02 (FT 1801, also coded as AL 104/LFI 6004), originally isolated from Atlantic salmon by staff of the Norwegian Veterinary Institute (Oslo, Norway), *Escherichia coli* (ATCC 25922), and the Gram-positive bacteria *Staphylococcus aureus* (ATCC 9144) and *Corynebacterium glutamicum* (ATCC 13032) were used as test organisms. All isolates were grown at room temperature in Mueller Hinton Broth (MHB; Difco Laboratories, Detroit, U.S.A).

### Extraction and Separation of Antibacterial Factors

Freeze-dried haemocytes (54.7 g) were extracted with 10 volumes (v/w) of 60% (v/v) acetonitrile (ACN; HPLC-grade, SDS, Peypin, France) containing 0.1% trifluoroacetic acid (TFA; Fluka Chemie AG, Buchs, Switzerland) for 24 hours at 4°C. The supernatant was collected, stored at 4 °C, and the residue was extracted once again (24 hours at 4 °C) under the same conditions. Due to high salt content (from the sample) and high acetonitrile content, the extraction medium solubilizes peptides but precipitates most higher molecular weight proteins and inhibits most proteolytic enzymes. The combined supernatants were incubated at -20°C for 1-2 hours to allow an organic (on top) and an aqueous phase to be partitioned. The aqueous phase was collected, dried in a vacuum centrifuge (Maxi Dry Lyo, Heto Lab., Denmark) and kept frozen at -20°C until further extraction.

The material was solubilized in 0.05% TFA to a concentration of 100 mg ml⁻¹. Salt was removed from the extract by solid phase extraction (SPE) as described by Haug et al. (2002a). Briefly, the extract was loaded onto a 35 cc Sep-Pak C₁₈ Vac cartridge (Waters Associates, MA, USA) equilibrated in acidified (0.05% TFA) Milli-Q water (Millipore Corp., MA, USA). After washing with acidified water (0.05% TFA), three stepwise elutions were performed with 10, 40, and 80% ACN in acidified water, respectively. Non-bound material was discarded. The different fractions collected were dried under vacuum and kept frozen at -20°C until subjection to reversed-phase high performance liquid chromatography (RP-HPLC). The protein content was determined by the BCA protein assay (Pierce, Rockford, IL, U.S.A) with bovine serum albumin as standard.

### Bioactivity of SPE Extracts

After solid phase extraction, all samples were diluted in water to a protein concentration of 250 µg/ml and serial two-fold dilutions were made before antibacterial activity testing. The test was performed in 96-well microtitre plates, as described previously (Haug *et al.,* 2002a). Briefly, 50 µl of test fractions were incubated with 50 µl of a suspension of an actively growing culture of bacteria diluted to a starting concentration of 5 x 10³ cells per well. Bacterial growth was assayed every 6 h by measurement of the optical density at 600 nm using a microtitre plate reader. Cecropin P1 (0.5 µg/ml) was used as a positive control. Antibacterial activity was determined when the optical density of the growth control (bacteria plus water) reached an absorbance at 600 nm of 0.15-0.20. Fractions were regarded as active when the optical density was less than 50% of the control, and fractions that were active at protein concentrations of 31.25 µg/ml or lower were considered as fractions with high activity. The results show that *in vitro* antibacterial activity is found in 40 and 80% SPE fractions of the coelomocytes (Table 2). Both fractions showed high activity, especially against *V. anguillarum*.

SPE fractions showing antibacterial activity were tested for proteinase K sensitivity, according to the method by Haug *et al.* (2002a). When the antibacterial activity was reduced by more than 50%, the fraction was regarded as proteinase K sensitive. In the same assay, proteinase K was inactivated by heat treatment (85° C, 15 min). As a control the fractions were subjected to heat treatment alone. Fractions having reduced activity after both proteinase K and heat treatment were regarded as heat sensitive. The results show that the activity in these fractions disappeared after treatment with proteinase K. None of the active fractions were sensitive to heat treatment. (Table 2).

Further, all SPE fractions were tested for lysozyme-like activity according to the method of Nilsen *et al.* (1999), with minor modifications. A standard suspension of *Micrococcus luteus* (Sigma Chemical Co. no. M-3770, St. Louis, MO, U.S.A) cell walls (0.3 mg/ml) was prepared in 100 mM sodium acetate (pH 5.2) and 100 mM NaCl (1:1), giving an ionic strength of 0.1. The test fractions were diluted to a protein concentration of 500 µg/ml. Experimental conditions consisted of 0.1 ml sample (less if high activity was detected) added to 3 ml of *M. luteus* suspension, and the reaction was carried out in a 1 cm wide cuvette at room temperature. The activity was determined spectrophotometrically (Hitachi U 2000, Tokyo, Japan) from the first minute of linear decrease in absorbance at 450 nm. Enzyme activity was expressed as units where one unit represents a decrease in absorbance of 0.001 per min. Lysozyme-like activity was judged to be present when enzyme activity was more than 20 units/mg protein. No lysozyme-like activity was detected in any of the sea urchin extracts.

To test whether the coelomocyte SPE fractions contain factors that display toxic effect on eukaryotic cells, the haemolytic activity was determined. The assay was performed as described by Haug *et al.* (2002a). Briefly, heparinased human red blood cells (RBC) were resuspended in PBS to a haematocrit value of 10 %. Samples were diluted to a protein concentration of 500 µg/ml and the test was performed in 96 well U-shaped microtitre plates. The test mixture consisted of 40 µl PBS, 50 µl test fraction and 10 µl of the RBC suspension. After incubation at 37°C for 1 h the plates were centrifuged at 200 x *g* for 5 min. The supernatants (60 µl) were transferred to new flat-bottomed polycarbonate microtitre plates and the absorbance was measured at 550 nm. Baseline haemolysis and 100 % haemolysis were defined as the amount of haemoglobin released in the presence of PBS and 0.1 % Triton X-100 (Sigma), respectively. Some haemolytic activity (10-30%) was found in the 40 and 80% SPE fractions.

### Purification of Antibacterial Peptides

The HPLC system (Waters Associates, Millipore Corp., MA, USA) consisted of a 600E pump, an in-line degasser AF, a Guard-Pak (4.0 µm I.D.) pre-column, a 717 autosampler, and a 2996 photodiode array detector. The absorbance (200-400 nm) was recorded using the Millenium v4.0 (Waters) software program. The HPLC system was operated at 25°C.

Based on the results from the bioactivity studies, we focused our attention on the 40% SPE fraction.

Step 1: The lyophilised 40% fraction from SPE was resuspended in 0.065% trifluoroacetic acid and subjected to RP-HPLC on a SymmetryPrep C₈ (Waters; 90Å; 7 µm; 7.8 x 150 mm) column. The mobile phase consisted of A) 80% acetonitrile in 0.05% TFA and B) 0.065% TFA. Elution was performed with a linear gradient of 0-75% A for 120 min at a flow rate of 2 ml/min. Absorbance was monitored at 220 nm and the exact concentration (C%) of acetonitrile for the elution of each peak was noted. Fractions were collected manually, dried under vacuum, reconstituted with Milli-Q water, and aliquots (50 µl) of the fractions were tested for antibacterial activity against *L. anguillarum* and *C. glutamicum*. Compounds with growth-inhibiting activity against both bacteria were eluted in a series of fractions from 20 to 45% acetonitrile. Of the two bacteria tested, *C. glutamicum* was the most sensitive.

Step 2: Four of the active fractions (Fig. 2, peaks 17, 18, 19 and 20) were resuspended in 0.065% trifluoroacetic acid (TFA) and subjected to RP-HPLC on a Symmetry Shield RP₁₈ (Waters; 90Å; 5 µm; 4.6 x 250 mm) column. The mobile phase consisted of the same ingredients as described above and elution was performed with linear gradients of 0.25% acetonitrile/min at a flow rate of 1 ml/min. The acetonitrile concentration at the start of the gradient was 5% lower than the C% determined in step 1. Fractions were hand collected and tested for antibacterial activity.

The antibacterial activities of the obtained HPLC fractions were determined by continuous monitoring of bacterial growth with a Bioscreen C microbiology reader (Labsystems Oy, Helsinki, Finland). The test was performed in 100-well flat-bottomed honeycomb plates, in which 50 µl of test fractions were incubated with 50 µl of a suspension of an actively growing culture of bacteria diluted to a starting concentration of 5 x 10³ cells per well. The growth chamber was maintained at 20°C during the incubation period. The absorbance was measured at 2 h intervals for 48 h by a turbidometric method with vertical light photometry and a wide band filter (420-580 nm), which is more insensitive to colour change in the sample compared to single wavelength detection. Cecropin P1 (0.5 µg/ml) was used as a positive control during these experiments. Antibacterial activity was determined when the optical density (OD) of the growth control (bacteria plus water) reached an absorbance of approximately 0.3. Fractions were regarded as active when the OD was less than 50% of the growth control.

Finally, four peptides (P 17, P 18, P 19 and P20) corresponding to the active fractions were purified to homogeneity as monitored by RP-HPLC and ESI-MS (Mass spectrometry analysis is described in detail in the next section). These peptides were submitted to further chemical characterization.

Step 3: Non-pure, active fractions were re-chromatographed on a Symmetry 300 C₁₈ column (Waters; 300Å; 5 µm; 4.6 x 250 mm) and eluted under the same experimental conditions as described in step 2, but with a flow rate of 0.5 ml/min. Fractions were collected manually and submitted to mass spectrometry.

### Mass Spectrometry Analysis

Electrospray ionization mass spectrometry (ESI-MS) was performed with a Quattro-LC triple quadrupole instrument equipped with an electrospray LC interface (Micromass UK Ltd., Wythenshawe, UK). The analysis was performed in ESI⁺ mode with a cone voltage of 40 V. Samples were dissolved in 95% (v/v) methanol containing 0.02% formic acid and were pumped (Pump 11, Harvard Apparatus, Holliston, MA, USA) into the mass spectrometer at a flow rate of 10 µl min-1. N₂ was used as desolvation (flow: 300 1h⁻¹) and cone gas (flow: 401 h⁻¹), and the temperature in the ionization chamber was 100°C. The quadrupole was scanned from m/z 200 to 2000 at 10 s/scan and the ion signals were recorded using the MassLynx™ v4.0 (Micromass) software program. The data were recorded in the continuum mode of acquisition. Non-protonated monoisotopic molecular masses were calculated from a series of multiple-charged protonated molecular ions.

### Amino Acid Analysis and Sequencing

The purified peptides were hydrolysed in vacuum in 6 M HCl for 20 h at 110° C. Amino acids were derivatized with phenylisothiocyanate and the corresponding phenylthiocarbamyl residues were subsequently separated and analysed on an automatic amino acid analyser (Model 421, Applied Biosystems, Perkin Elmer) calibrated with 100 pmol of phenylthiohydantoin amino acid standards.

Edman degradation of native peptides was performed at the Biotechnology Centre of Oslo (University of Oslo, Norway), and was performed on a protein microsequencer model 477A with120A PTH analyser (Applied Biosystems, Perkin Elmer) and a HP 241 Protein Sequencer (Hewlett-Packard).

Reduction/alkylation of P17 and P20, proteinase digestion, purification of fragments by RP-HPLC, automatic Edman degradation of alkylated peptides, and electrospray ionization mass spectrometry (ESI-MS) were performed at Eurosequence B.V. (Groningen, The Netherlands, http://www.eurosequence.nl/). A portion of P17 was subjected to alkylation in the gas phase using 4-vinylpyridine, and further sequenced by Edman degradation. Since the pyridylethylated fragments of P 17 were not easily separated by HPLC, it was decided to alkylate the peptide with iodoacetamide, which converts Cys into carboxyamido-methylcysteine (CAM-Cys). Next, the alkylated sample was subjected to digestion with Eridoproteinase Lys-C (ELC), an enzyme which cleaves at the carboxyl end of Lysine residues. The resulting digest was separated by HPLC and analysed by Edman degradation. A portion of P20 was subjected to alkylation using iodoacetamide. The heavy and light chain were separated by HPLC and subjected to Edman degradation.

### Sequence Analyses

Homology searches were performed against the SwissProt, NR, PAT and Month databases with the Basic Local Alignment Search Tool (BLAST) (Altschul et al., 1997) provided by the NCBI server (http://www.ncbi.nlm.nih.gov/BLAST). Homology searches of full and partial sequences were also performed against the Antimicrobial Peptide Database (http://aps.unmc.edu/AP/main.html) (Wang and Wang, 2004) and against ANTIMIC (http://research.i2r.a-star.edu.sg/Templar/DB/ANTIMIC/), a database of antimicrobial peptide sequences (Brahmachary et al., 2004). Peptide mass, amino acid composition, isoelectric point, and sequence alignment were predicted by the Expert Protein Analysis System (ExPASy) proteomics server of the Swiss Institute of Bioinformatics (http://www.expasy.ch/). Secondary structure was predicted using methods provided by The PSIPRED Protein Structure Prediction Server (http://bioinf.cs.ucl.ac.uk/psipred/) (McGuffin et al., 2000).

### Primary Structure Determination

The six peptides purified from coelomocytes were separately analysed for their primary structure. Mass measurement by ESI-MS showed average masses ranging from 4.4-4.8 kDa (Table 6). Distinct UV absorbance at 280 nm supports that all these components contain tyrosine and/or tryptophan residues.

Amino acid analysis of P17 and P20 (Table 3) revealed differences in the amino acid content between some of the peptides. P 17 and P20 are both characterised by a high content of glycine and alanine. Furthermore, P20 seem to be low in content or devoid of tyrosine and proline.

NH₂-terminal sequence analysis of P17-P20 (Table 5) revealed several amino acids in each position, indicating that these peptides were composed of more than one peptide chain. Two of the peptides were subjected to alkylation and Endoproteinase Lys-C treatment, followed by RP-HPLC purification of peptide fragments and Edman degradation. Several fragments were obtained from P17, and by peptide mapping, two peptide chains (one heavy chain and one light chain) were identified (Fig 4a). Both peptide chains contained one cysteine residue, which may form a disulfide bridge, thereby making a dimeric structure of the mature peptide.

The fragment from P17 having the sequence of SGIHAGQRGCSALGF was further analysed by Nanospray ESI-MS, in order to establish its exact mass, and in order to investigate if C-terminal residue of the heavy chain is indeed phenylalanine. The observed mass (doubly charged m/z ion at 759.38; spectre not shown) is consistent with the C-terminal Phe-containing peptide structure (monoisotopic mass: 1517 Da), therefore unambiguously determining the structure of this Lys-C peptide, originating from the C-terminal pert of the heavy chain of P17.

The heavy chain contains an unknown (probably post-ribosomally modified) residue at position 2. An N-terminal fragment having the sequence XXFKKTFHKVSHAVKS was also characterised after proteinase treatment. Obviously, the lysine residues were not cleaved by Endoproteinase Lys-C. Most likely, the unknown residue and perhaps somewhat bulky residue at position 2 prohibits efficient cleavage of the Lys residues. The calculated mass of the dimeric peptide (without the unknown residue) is 4222.86 Da, some 265.44 Da short of the measured mass (4488.30 Da). The missing mass is close to a brominated tryptophan residue, having a mass of 265.11 Da.

P20 was alkylated, followed by RP-HPLC purification of peptide chains and Edman degradation of each chain. Both peptide chains contained one cysteine residue, which together may form a disulfide bridge, thereby making a dimeric structure of the mature peptide.

The heavy chain of P20 contains, similar to P 17, an unknown (probably post-ribosomally modified) residue at position 2.

Based on UV-Vis absorbance at 280 and 297 (supports tryptophan) of N-terminal peptide fragments, amino acid analysis, and similarity to protein sequences from *Strongylocentrotus purpuratus*, the unknown residue in P 17 and P20 is very likely a modified tryptophan. By comparing measured masses (mass spectrometry) on intact, native peptides and calculated masses of the peptide chains, the modification represents a mass difference of 80±1 Da. This mass difference could, according to ExPASy amino acid modification page (bttp://au.expasy.org/tools/findmod/findmod_masses.html), be due to bromination, phosporylation or sulfation. Tryptophan lacks available hydroxyl-groups (needed for sulfation or phosporylation) in its chemical structure, which supports that the modification is a brominated tryptophan.

### Genomic DNA and RNA isolation

Genomic DNA was extracted from coelomocytes using Blood & Cell culture DNA mini kit, QIAGEN (Hilden, Germany) according to the manufacture's instructions. Total RNA was isolated from the pellets of whole coelomic fluid by the QIAZol^{™} reagent from QIAGEN (Maryland, USA) according to the manufacturer's instructions. Messenger RNA was purified from extracted total RNA by kit (Oligo tex RNA midi kit, QIAGEN, Hilden, Gemany) according to the manufacturer's instructions. The concentration and quality of DNA, total RNA and mRNA were measured by Nano-drop ND-1000 spectrophotometer (Wilmington, DE, USA).

### Reverse transcription-polymerase chain reaction (RT-PCR) and degenerate oligonucleotide primed PCR (DOP-PCR)

Messenger RNA was isolated as described above from coelomocytes. RT-PCR reaction was performed using kit (Tagman® reverse transcription reagent Applied Biosystems, New Jersey, USA). Briefly, the following was added to a reaction: 5 µl 10 × reaction buffer, 70 ng mRNA, 2µl (400 ng) modified oligo (dT) primer (G413), 1.25 µl Reverse transcriptase and 1 µl RNase inhibitor, 11 µl 25mM MgCl₂, 1 µl dNTP10 mM, and water to bring the reaction volume up to 50 µl. The reverse transcription reaction was conducted in a thermocycler with the following thermo-profiles: 25°C for 10 min, 48°C for 45 min and 95°C for 5 min for one cycle.

DOP-PCR was performed on a thermocycler in two separated steps using 2 µl first strand cDNA as a template, 5 µl 10 × Optimized DyNAzyme™ Buffer, 2 µl forward degenerate primer, 0.5 µl reverse primer (G479), 1 µl dNTP10mM, 0.2 µl DyNAzyme™ II DNA (Finnzymes, Espoo, Finland) and water to bring the reaction volume up to 50 µl. In the first phase, the DOP-PCR was carried out using 94 °C for 5 min, 7 cycles at 94 °C for 25 seconds min, 42°C for 25 seconds and 72 °C for 1 min. The second phase was completed with 30 cycles of 94 °C for 25 seconds min, 55°C for 25 seconds and 72 °C for 1 min, followed by a final extension at 72 °C for 10 min. The DOP-PCR products were analyzed by electrophoresis on a 1.8 % agarose gel and documented with Bioimaging system, Syngene.

The product of DOP-PCR was gel-purified on a 1.0 % agarose gel, extracted using a Gel extraction Kit (OMEGA EZNA^{™}, Duraville, USA) and cloned into pGEM®-T Easy Vector Systems (Promega, Madison, WI, USA). Transformants were selected on LB agar containing 100 µg of ampicillin/ml and 40 µg of 5-bromo-4-chloro-3-indolyl-β-D-galactopyranoside (X-gal)/ml. Plasmid DNA from white colonies was isolated using the plasmid mini prep kit (OMEGA EZNA^{™}, Duraville, USA). The universal primers SP6 and T7 were employed to sequence the plasmid inserts using Applied Biosystems Big-Dye^{™} version 3.1 kit at 1/4th the manufactures recommended scale. Sequence data were collected on an Applied Biosystems model 3730 automated capillary DNA sequencer. The plasmid containing SD 16 insert was named as pSD 16. The amino acid sequence of SD16 was deduced and aligned with Edman degradation sequence using BioEdit Sequence Alignment Editor Version 7.0.5.2 software.

Based on these results, the primers 147F-SD and 147R-SD were designed for studying the gene structure. Genomic DNA was employed as a template in PCR (30cycles, 94 °C for 30 seconds min, 60°C for 30 seconds and 72 °C for 90 seconds).

### cDNA library construction and screening

The ZAP-cDNA^{®} Synthesis Kit and ZAP-cDNA^{®} Gigapack^{®} III Gold Cloning Kit (Stratagene) was used to create coelomic cDNA libraries according to the manufacture's instructions. After amplifying step, the cDNA library was stored in 7% dimethyl sulfoxide at -80°C.

The 25ng SD16 DNA fragment from pSD16 was templated to amplify the ³²P labelling probes using Redi prime^{™} II, Random prime labelling system (Amercahm Biosciencespcr) according to the manufacturer's instructions. After plating and growing of the phages 5x10⁴ pfu/plate at 37°C for 8 hours, the plaque were lifted in duplicate onto a nitrocellulose(+) membrane (Amersham Biosciences). Membranes were submerged into denaturation buffer (0.1 M NaOH, 1.5 M NaCl) for 1.5 minutes; neutralization buffer (0.2 M Tris-HCl, pH 7.4) for 5 minutes; and for 2 minute in saline sodium citrate buffer (SSC) 2x, pH 7.0. DNA was cross-linked by oven baking at 80°C for 2 hours. Hybridization was performed for 12 hours at 65°C. Then, membranes were washed twice at 55°C for 15 minutes each using low stringency solution (0.1%SDS, 2xSSC). Positive plaques were visualized by exposure with Biomax MS film (Kodak, USA). Positive clones were purified by isolation of the plaque and two additional screening procedures. After the third screening, positive clones were excised into plasmid associated with helper phage. Plasmids pBluescriptSK(-), containing the target inserts, were sequenced using primer T3 and T7, as described before.

### Isolation of the polypeptides of the present invention from other organisms.

Obtaining the native sequence of the peptides P17 and P20 is significant for purposes other than the synthesis of the "Strongylosin" per se: the availability of the naturally occurring sequences provides a useful probe to obtain corresponding DNA encoding P17 and P20 of other species. Thus, cDNA libraries, for example, of coelomocytes derived from other echinoderms can be screened using the native DNA, preferably under conditions af high stringency. High stringency is as defined by Molecular cloning: a laboratory Manual 3 ed, Cold Spring Harbor Laboratories Press (2001), the relevant portions of which are incorporated herein. This procedure also permits recovery of allelic variants of these peptides from the same species.

Alternatively, P17 and P20 homologs are isolated from coelomocytes of a desired species using techniques as described for *S. droebachiensis* above. The location of fragments having antimicrobial activity corresponding to P 17 and P20 are assessed using criteria of molecular weight and assaying the fractions for the desired activities as described herein.

### cDNA sequence of P17, P18, P19 and P20

Total RNA is extracted from the coelomocytes of the green sea urchin *S. droebachiensis* with Trizol. Common techniques known to persons skilled in the field are used. cDNA is made from mRNA. The full length cDNA sequences were cloned by using 5' and 3' rapid amplification of cDNA ends (5'- and 3'-RACE) PCR approaches. The universal primers are from SMART^{™} RACE cDNA amplification kit (Clontech, US) and gene specific primers are based on the *S. droebachiensis* antimicrobial peptides described herein. The isolated P17, P 18, P19 and P20 cDNAs are automatically sequenced. The complete cDNA sequences were obtained by overlapping the fragments. All methods employed are known to the skilled artisan.

### Peptide synthesis (P17 fragments)

Brominated (containing 5-Bromo-D/L-tryptophan) and non-brominated fragments of P17 (table 8) were synthesized at BIOMOL International, LP (Exeter, UK). The amino acids 5-Bromo-D/L-tryptophan and 6-Bromo-D/L-tryptophan were purchased from Biosynth AG (Staad, Switzerland).

### EXAMPLES

The following examples are meant to illustrate how to make and use the invention. They are not intended to limit the scope of the invention in any manner or to any degree.

### Example 1

### Antibacterial activity (P17, P19, P20)

The polypeptides of the present invention were isolated and subsequently tested for activity. A serial dilution method was employed to examine the antibacterial activity of the peptides. Two Gram-negative bacteria, *L. anguillarum* and *E. coli*, and two Gram-positive bacteria, *C. glutamicum* and *S. aureus,* were chosen as test bacteria. The polypeptides of the present invention were active against both Gram-positive and Gram-negative bacteria (table 4).

### Example 2

### Antibacterial activity (Brominated and non-brominated fragments of P17)

The antibacterial activities of the different peptides were determined by continuous monitoring of bacterial growth with a Bioscreen C microbiology reader (Labsystems Oy, Helsinki, Finland). The test was performed in 100-well flat-bottomed honeycomb plates, in which 50 µl of test fractions were incubated with 50 µl of a suspension of an actively growing culture of bacteria diluted in Mueller Hinton medium to a starting concentration of 3 x 10⁴ cells per ml. The Gram-negative bacteria, *Listonella (Vibrio) anguillarum*, serotype 02 (FT 1801, a fish pathogenic strain), *Pseudomonas aeruginosa* and *E. coli* (ATCC 25922), and the Gram-positive bacteria, *C. glutamicum* (ATCC 13032) and *S. aureus* (ATCC 9144), were chosen as test bacteria. The growth chamber was maintained at either 20 or 37 °C during the incubation period. The absorbance was measured at 2 h intervals for 48 h by a turbidometric method with vertical light photometry and a wide band filter (420-580 nm), which is more insensitive to color change in the sample compared to single wavelength detection. Antibacterial activity was determined when the optical density (OD) of the growth control (bacteria plus water) reached an absorbance of 0.3-0.4. Fractions were regarded as active when the OD was less than 50% of the growth control. Polymyxin B (Sigma, St. Louis, MO, USA), Lactoferricin B, Cecropin P1 and *Hyalophora cecropia* Cecropin B were used as reference. The results (table 9) show that several of the synthetic fragments of P 17 (brominated and non-brominated derivates of the heavy chain) display antibacterial activity.

The most active derivates of P 17 (brominated and non-brominated) were tested for their ability to inhibit bacterial growth and biofilm formation of clinical isolates, including *Pseudomonas* K15-60, *Acinetobacter baumannilii* K15-9, *Citrobacter freundii* K15-75, *Bacillus* K13-61, E. *coli* K15-58, *Klebsiella* K15-72 and *Salmonella* K14-40. The Antimicrobial molecules were also tested against drug resistant clinical isolates, such as the commonly encountered multi-resistant human pathogen *S. aureus* (MRSA) K15-64 and the glycopeptide (vancomycin)-resistant *Enterococccus faecium* (GREF) K9-28. The non-clinical isolates *Escherichia coli* (ATCC 25922), *Pseudomonas aeruginosa* (ATCC 27853), *Corynebacterium glutamicum* (ATCC 13032), and *Staphylococcus aureus* (ATCC 9144) were also tested for comparison. Serial dilutions of synthetic peptides were made in double distilled water and tested according to the method described above. However, in these experiments the starting concentration of bacteria were 3 x 10⁶ cells per ml and the temperature in the growth chamber was 37 °C. Polymyxin B was used as positive control for the Gram-negative bacteria. The results (table 10) show that P17 HC-Br, P 17 HC and P17HC (1-20) display antibacterial activity against various clinical isolates, including multiresistant bacteria (MRSA and GREF), at µM concentrations.

### Example 3

### Bacterial cell viability (Brominated and non-brominated fragments of P17)

To evaluate the effect of antimicrobial peptides on the viability of bacteria in a fast and reliable manner we used the *lux CDABE* operon from *Photorhabdus luminescens* as a reporter system in gram negative test bacteria and the modified *luxABCDE* operon in *S. aureus* (Vesterlund *et al.* 2004). Bacterial bioluminescence is directly dependent on metabolic activity, since reduction equivalents are needed to drive light emission. Any form of interruption of bacterial cell integrity or metabolism will therefore be detected as a reduction in light emission.

For the assay we used *S. aureus* (RN4220), *E. coli* K-12 and *Salmonella enterica* serovar typhimurium (ATCC 14028) transformed with the respective lux-operon containing plasmids (Vesterlund *et al.* 2004).

For each experiment a fresh colony was picked from Luria-Bertani plates supplemented with 10 µg/ml of erythromycin in case of *S. aureus* and with 100 µg/ml of ampicillin in case of *E. coli* and *S. enterica* serovar Typhimurium. Each colony was suspended in 5 ml of Müller-Hinton medium with appropriate antibiotics. The culture was incubated over night at 37°C in a shaking incubator. The next morning 0.5 ml of this culture was transferred to 30 ml fresh MH medium in an Erlenmeyer-flask supplemented with the respective antibiotic. The flask was incubated (200 rpm shaking; 37°C) until an OD₆₀₀ of 0.5-0.8 was reached. The culture was then diluted to an OD₆₀₀ of about 0.02-0.03 before transfer to the 96 well opaque plates with optical bottom (Packard ViewPlate) used for the assay.

The synthesized peptides were dissolved in water and aliquots frozen at -18°C until use. Twofold dilutions of test peptides (final concentrations ranging from 0.31 to 80 µM) were directly prepared in 96 well microtiter plates in a volume of 10 µl in water. 90 µl of indicator bacteria in MH medium were added directly to the prepared and prewarmed (30 minutes at 37°C) plate before starting the measurement in a Victor multiplate reader (Perkin Elmer) at 37°C. Both OD₆₀₀ and luminescence in counts per second (cps) were followed over 5 hours in measuring intervals of 5 minutes. The plates where shaken before each measurement (20 sec.; 5mm orbital shaking; 10 sec waiting). The growth curves and light emission curves were drawn and evaluated to determine the MIC for each indicator strain. The MIC was defined as the concentration of test compound causing more than 50% reduction in bioluminescence (cps) or optical density (OD₆₀₀) after 5 hours, compared to the growth control. Cecropin P1 was used as positive control. The results (table 14a-b) confirm the results obtained in example 2, showing a pronounced antibacterial effect of the longest peptide fragments. The luminescence assay shows that both full length HC peptides (P17 HC and P17 HC-Br) reduce bacterial viability already after very short incubation times. The antibacterial effect seems to be independent of the tryptophan modification (bromidation) since P17 HC and P17 HC (1-20) and their brominated counterparts were equally active. The partial peptides P17 HC (1-20), both bromated and unbromated, inhibit growth only in higher concentrations. This indicates that the amino acids 21-30, cut off in P17 HC (1-20), are responsible for a 30 times increase in antibacterial activity, although the partial peptide 21-30 in itself did not show antimicrobial activity in the concentrations tested (see table 14).

In a similar experiment, the culture was diluted to an OD600 of about 0.25-0.30 before addition of peptides, and the OD₆₀₀ and luminescence were followed over 3 hours in measuring intervals of 5 minutes. The luminescence assay shows that a full bactericidal effect is reached already after 15 minutes of incubation with the full length HC peptides (P 17 HC and P 17 HC-Br), at the highest concentration (80 µM) tested (figure 11). This bactericidal effect seems to be concentration dependent. In this study, the bromidation seems to alter the activity of the peptides and make them more active.

### Example 4

### Effect of bacterial LPS on antibacterial activity - indication of LPS binding (Brominated and non-brominated fragments of P17)

A second plate with an otherwise similar setup (as described for the luminescence assay in example 3) was prepared, but with addition of *Aeromonas salmonicida* LPS to the peptide dilutions before pre-warming. The LPS end concentration in each well was approximately 2.5 µM. The results show (Table 14a and b) that the antibacterial activity of the peptides tend to decrease when coincubated with LPS (especially for the Gram-negative bacteria), indicating that the active peptides have LPS (endotoxin)-binding activities.

### Example 5

### Antifungal activity (Brominated and non-brominated fragments of P17)

The various derivates of P 17 (brominated and non-brominated) were tested for their ability to inhibit fungal growth of the yeast strains *Saccaharomyces cerevisiae* sp, *Candida albicans* (ATCC 10231) and *Rodotorula sp.*, and the filamentous strains *Botrytis cinerea* 101, *Penicillium rogveforti*, and *Fusarium sp.* All strains, except *C. albicans*, were a gift from Prof. A. Tronsmo (The Norwegian University of Life Sciences, Ås, Norway). The fungi were cultivated on potato dextrose agar, supplemented with 2% glucose, at room temperature. Before antifungal testing, the fungi were dissolved in potato dextrose broth (Difco) at half-strength. Cell concentration was determined and adjusted after counting in a Bürker chamber. The filamentous fungi were filtered through a piece of cotton before counting. An aliquot of 50 µl of fungal spores (final concentration 2 x 10⁵ spores per ml) were inoculated in microtiter plates (96 wells) along with 50 µl peptide solution. Peptides, dissolved in distilled water, were tested at final concentrations (two-fold dilutions) ranging from 50 µM to 0.1 µM. Cultures were grown in the dark (without shaking) and with a moist chamber at 25°C (37°C for *C. albicans*). Growth inhibition was determined microscopically after 48 h of incubation. MIC was determined as the lowest concentration of peptide resulting in 50% inhibition of visible growth. The results (table 11) show that several derivates (brominated and non-brominated) of P 17 display antifungal activity, with P 17 HC and P17 HC-Br being most potent.

### Example 6

### Antitumoral activity (Brominated and non-brominated fragments of P17)

The cytotoxic effects of the peptides on different human tumour cells were measured using the MTT colorimetric assay (Mosmann, 1983). MTT (3-(4-5-dimethylthiazol-2-yl)-2,5-diphenyl tetrazolium bromide) is a soluble tetrazolium salt yielding a yellow solution when prepared in media or salt solutions lacking phenol red. Dissolved MTT is converted to an insoluble purple formazan by cleavage of the tetrazolium ring by dehydrogenase enzymes. This water insoluble formazan can be solubilized using isopropanol or other solvents and the dissolved material is measured spectrophotometrically. The absorbance was measured as a function of concentration of converted dye. The conversion of the soluble dye to the insoluble purple formazan is utilized in assays for measurement of cell proliferation. Active mitochondrial dehydrogenases of living cells cause this change, while dead cells do not. We used this assay to measure the degree of cell death caused by the peptides.

### Materials:

MTT and culture media were obtained from Sigma-Aldrich AS (Oslo, Norway). Fetal bovine serum (FBS) was purchased from Biochrom, KG (Berlin, Germany). L-glutamine and L-glutamine-L-alanine (Glutamax) were purchased from Gibco (Paisley, UK). The human breast malignant carcinoma cell line (MT-1) and the human skin malignant melanoma cell line (FemX) were provided by Dr. Ø. Fodstad, Department of Tumour Biology, the Norwegian Radium Hospital, Oslo, Norway. The human colorectal adenocarcinoma cell line HT-29 (HTB-38), the human skin malignant melanoma cell line A2058 (CRL-11147), the murine colon carcinoma cell line CT26 (CRL-2638), the murine B lymphoma (reticulum cell sarcoma) cell line A20 (TIB-208), and the human embryonic lung fibroblast cell line MRC-5 (CCL-171) were all obtained from the American Type Culture Collection (ATCC). The human breast malignant carcinoma cell line MCF-7 (HTB-22) was obtained from the European Collection of Cell Cultures (ECACC).

### Experiment 1. Activities against the human tumor cell lines FemX, HT-29 and MT-1. Cell cultures:

The tumour cell lines FemX, HT-29, MT-1 were maintained in RPMI-1640 medium supplemented with 10% heat inactivated FBS and 1% L-glutamine in a humidified atmosphere of 5% CO₂ at 37°C. The human embryonic fibroblast cell line, MRC-5 (which is normal lung fibroblasts), was cultured as a monolayer in MEM medium, under the same conditions as described for the tumour cell lines. Cells to be used in the assay were grown to a minimum of 75% confluency, trypsinated and split to single cell suspension, counted and centrifuged at 1500 rpm for 10 min. The cell pellet was resuspended to a concentration of 4 x 10⁵ cells/ml (HT-29) or 2 x 10⁵ (MT-1, MRC-5 and FemX) in RPMI-1640 without FBS and L-glutamine (assay-medium).

### In vitro cytotoxicity:

The cytotoxicity was measured using the MTT method (Mosmann, 1983), with minor modifications. Different human tumour cells (2-4 x 10⁵) were applied in 96-well culture plates in a volume of 100 µl RPMI 1640 medium and incubated at 37°C for 24 hours. The medium was then removed and the cells were added 50 µl fresh medium and treated with 50 µl of peptide solution (P17-HC, P 17-HC-Br, P17-LC, concentrations ranging from 0 to 200 µg/ml), diluted in H₂O, and the plates were incubated at 37°C for 24 hours. Cells treated with Triton X-100 (1%) were used as positive control (100% killing) whereas cells plus medium only were used as negative control. Following the incubation period, 10 µl MTT (5 mg/ml) dissolved in phosphate-buffered saline (PBS) was added to each well, and the plate was further incubated for 2 hours. A volume of 70 µl of the supernatant was then removed and 100 µl acid alcohol (0.04 M HCl in isopropanol) added to each well to dissolve the dark blue crystals. The plate was placed on a shaker for 1 hour at room temperature and read spectrophotometrically at 590 nm in a microtiter plate reader (DTX880 Multimode Detector, Beckman Coulter). Cell survival (%) was then determined from the ΔA₅₉₀ nm relative to negative control (100% living cells) and expressed as 50% inhibitory concentration (IC₅₀). The IC₅₀ values were estimated from the dose-response curves, which were obtained by averaging at least 3 parallel experiments. The results (table 12a) show that P17 HC and P17 HC-Br display potent cytotoxic activity (IC₅₀<10 µM) against the human skin malignant melanoma cell line (FemX) and some activity against the human breast malignant carcinoma cell line (MT-1).

### Experiment 2. Activities against the human tumor cell lines MCF-7, A2058 and HT-29. Cell cultures:

MCF-7 was maintained in EMEM, A2058 in DMEM and HT-29 in RPMI-1640 medium supplemented with 10% heat inactivated FBS and 1% L-glutamine-L-alanine in a humidified atmosphere of 5% CO₂ at 37°C. The human embryonic fibroblast cell line, MRC-5 (which is normal fibroblasts), was cultured as a monolayer in MEM medium, under the same conditions as described for the tumour cell lines. Cells to be used in the assay were grown to a minimum of 75% confluency, trypsinated and split to single cell suspension, counted and centrifuged at 1500 rpm for 10 min. The cell pellet was resuspended to a concentration of 2 x 10⁵ cells/ml (HT-29) or 1 x 10⁵ (MRC-5, MCF7 and A2058) in DMEM without FBS and L-glutamine-L-alanine (assay-medium). All cell lines were routinely tested for mycoplasma infection using a PCR-based mycoplasma detection kit (MycoSensor PCR Assay Kit, Stratagene), following the supplier's instructions. All cell lines were mycoplasma free.

### In vitro cytotoxicity:

The cytotoxicity was measured using the MTT method (Mosmann, 1983), with minor modifications. Different human tumour cells (1-2 x 10⁵) were applied in 96-well culture plates in a volume of 100 µl DMEM medium and incubated at 37°C for 24 hours. The medium was removed and the cells were treated with 100 µl of peptide solution (concentrations ranging from 0 to 200 µg/ml), diluted in a) RPMI-1640 medium supplemented with 5% heat inactivated FBS, or b) RPMI-1640 medium without FBS.The plates were incubated at 37°C for 24 hours. Cells treated with Triton X-100 (1%) was used as positive control (100% killing) whereas cells plus medium only were used as negative control. Following the incubation period, 10 µl MTT (5 mg/ml) dissolved in PBS was added to each well, and the plate was further incubated for 2 hours. A volume of 70 µl of the supernatant was then removed and 100 µl acid alcohol (0.04 M HCl in isopropanol) added to each well. The plate was placed on a shaker for 1 hour at room temperature and read spectrophotometrically at 590 nm in a microtiter plate reader (DTX880 Multimode Detector, Beckman Coulter). Cell survival (%,averaging at least 3 parallels) and IC₅₀ values were estimated as described above. The results (table 12b and 12c) show that P 17 HC and P17 HC-Br display potent cytotoxic activity (IC₅₀<10 µM) against the human skin malignant melanoma cell line (A2058) and the human breast malignant carcinoma cell line (MCF-7).

### Experiment 3. Activities against the murine tumor cell lines CT26 and A20.

### Cell cultures:

CT26 and A20 were maintained in RPMI-1640 medium supplemented with 10% heat inactivated FBS and 1% L-glutamine in a humidified atmosphere of 5% CO₂ at 37°C. The human embryonic fibroblast cell line, MRC-5, was cultured as a monolayer in MEM medium, under the same conditions as described for the tumour cell lines. CT26 and MRC-5 cells to be used in the assay were grown to a minimum of 75% confluency, trypsinated and split to single cell suspension, counted and centrifuged at 1500 rpm for 7 min. The cell pellet was resuspended to a concentration of 1-2 x 10⁵ cells/ml in RPMI-1640 medium without FBS and L-glutamine-L-alanine (assay-medium). The A20 cells were grown in suspension and adjusted to a concentration of 6 x 10⁵ cells/ml in assay medium. All cell lines were routinely tested for mycoplasma infection using a PCR-based mycoplasma detection kit (MycoSensor PCR Assay Kit, Stratagene), following the supplier's instructions. All cell lines were mycoplasma free.

### In vitro cytotoxicity:

The cytotoxicity was measured using the MTT method (Mosmann, 1983), with minor modifications. The adherent cells (CT26 and MRC-5) were applied in 96-well culture plates in a volume of 100 µl assay medium and incubated at 37°C for 24 hours. The A20 cells were applied to the microtiter plates just prior to testing. The medium was removed and the cells were treated with 100 µl of peptide solution (concentrations ranging from 0 to 400 µg/ml), diluted in serum-free medium, and the plates were incubated at 37°C for 4 hours. Cells treated with Triton X-100 (1%) were used as positive control (100% killing) whereas cells plus medium only were used as negative control. Following the incubation period, 10 and 20 µl MTT (5 mg/ml, dissolved in PBS) were added to each well in the adherent cells (CT26 and MRC-5) and the A20 cells, respectively. After further incubation for 2 hours, a volume of 70 µl of the supernatant was removed and 100 µl acid alcohol (0.04 M HCl in isopropanol) added to each well in the adherent cell lines. For the cells grown in suspension (A20), a volume of 130 µl of the supernatant was removed and 100 µl acid alcohol was added. The plates were placed on a shaker for 1 hour at room temperature and read spectrophotometrically at 590 nm in a microtiter plate reader (Thermomax Molecular devices, New Jersey, USA). Cell survival (%, averaging at least 2 parallels) and IC₅₀ values were estimated as described above. The results are shown in table 12d.

### Example 7

### Antiinflammatory effects (Brominated and non-brominated fragments of P17)

### Materials:

Fetal bovine serum (FBS) was purchased from Biochrom, KG (Berlin, Germany). Phorbol 12-myristate 13-acetate (PMA), lipopolysaccharide (LPS; *Escherichia coli* 0111:B4), MTT, and cycloheximide were obtained from Sigma-Aldrich (Oslo, Norway). Human monocytic THP-1 cells were obtained from the American Type Culture Collection (ATCC, Rockville, MD, USA). RPMI-1640 media was obtained from Gibco BRL (Paisley, UK). All other chemicals were obtained from Sigma-Aldrich.

### Cell culture conditions:

The THP-1 cells (ATCC, TIB-202) were maintained in suspension in RPMI-1640 medium with 25 nM HEPES and 2mM Glutamax I supplemented with 10 % FBS (growth medium) in a humidified atmosphere of 5% CO₂ at 37°C. Cells to be used in the assay were grown to a density of 8 x 10⁵ - 1 x 10⁶ cells/ml. The cells were centrifuged for 7 min at 200 g and the cell pellet was resuspended to a concentration of 10⁶ cells/ml in RPMI-1640 with 10 % FBS.

### Induction and analysis of TNF-α levels:

To allow examination of antiinflammatory effects of the peptides on basal and stimulated cytokine production, peptide-treated macrophages were cultured in the presence of LPS and the levels of TNF-α were measured. The primary screening was based on a mammalian cell line; the human THP-1 line. This line is of monocyte/macrophage origin and responds to various stimuli by increased production of cytokines, like TNF-α and the IL-1β, (Wang et al. 2000). To induce monocyte-macrophage differentiation, THP-1 cells were cultured in 96-well culture plates (10⁶ cells/well) in a volume of 100 µl RPMI 1640 medium, containing 50 ng/ml PMA (which is known to induce the maturation of monocytes) and incubated in a humidified atmosphere of 5% CO₂ at 37°C for 48 hours. The medium was then removed and the cells were washed with PBS, added 100 µl fresh medium (without PMA) and incubated for another 24 hours. Medium was removed and the adherent macrophages were treated with 100 µl of peptide solution (concentrations ranging from 0 to 400 µg/ml), diluted in RPMI-1640 medium with 25 mM HEPES and 2mM Glutamax I, but without FBS (assay medium). After incubation at 37°C for 1 hour, the cells were added LPS (0.1 µg/ml) and further incubated for 6 hours. Cycloheximide (Chx, 10 µg/ml) was used as a an inhibitory control for LPS induced TNF-α, whereas PUMA (50 ng/ml) and LPS (0.1 µg/ml) were used as controls for induction TNF-α. RPMI media was used as negative, unstimulated control. After incubation, the media (supernatant) was transferred to new 96-well culture plates and kept frozen (-20°C) until analysis. TNF-α concentrations were quantified by the Human TNF-α ELISA MAX kit (BioLegend), according to the manufacturer's instructions. Duplicate readings for each standard, control and sample were recorded. The average absorbance for each set of standards, controls, and samples was calculated using a standard curve. Concentrations were determined by extrapolation on the standard curve. The results (table 13 and figure 12) show that P 17 HC and P 17 HC-Br inhibits LPS induced TNF-α.resonse at low µM concentrations.

### Toxicity to THP-1 cells:

The toxicity of test peptides on THP-1 cells was measured using the MTT method (Mosmann, 1983), with minor modifications. To induce monocyte-macrophage differentiation, THP-1 cells were cultured in 96-well culture plates (10⁶ cells/well) in a volume of 100 µl RPMI 1640 medium, containing 50 ng/ml PMA (which is known to induce the maturation of monocytes) and incubated in a humidified atmosphere of 5% CO₂ at 37°C for 48 hours. The medium was then removed and the cells were washed with PBS, added 100 µl fresh medium (without PMA) and incubated for another 24 hours. Medium was removed and the adherent macrophages were treated with 100 µl of peptide solution (concentrations ranging from 0 to 800 µg/ml), diluted in RPMI-1640 assay medium without FBS. After incubation at 37°C for 1 hour, the cells were added LPS (0.1 µg/ml) and further incubated for 4 hours. Cells treated with Triton X-100 (1%) were used as positive control (100% killing) whereas cells plus medium only were used as negative control. Following the incubation period, 10 µl MTT (5 mg/ml) dissolved in phosphate-buffered saline (PBS) was added to each well, and the plate was further incubated for 2 hours. A volume of 70 µl of the supernatant was then removed and 100 µl acid alcohol (0.04 M HCl in isopropanol) added to each well to dissolve the dark blue crystals. The plate was placed on a shaker for 1 hour at room temperature and read spectrophotometrically at 595 nm in a microtiter plate reader (DTX880 Multimode Detector, Beckman Coulter). Cell survival (%) was then determined from the ΔA595 nm relative to negative control (100% living cells) and expressed as 50% inhibitory concentration (IC50). The IC50 values were estimated from the dose-response curves, which were obtained by averaging at least 3 parallel experiments. The results are shown in table 13.

### Example 8

### Effects on tissue factor response

### (Brominated and non-brominated fragments of P17)

### Induction and analysis of tissue factor (TF) levels:

A "whole blood" model was used for secondary screening. Venous blood from a healthy volunteer was drawn into polycarbonate tubes (Falcon; BD Biosciences Pharmingen, Franklin Lakes, NJ, USA) containing heparin (Sigma-Aldrich, Germany) in a final concentration of 10 U/ml. Blood sampling was performed under the protocols approved by the Regional Committee for Medical Research Ethics. Aliquots of 1 ml whole blood were incubated with test peptide (concentrations ranging from 0.25-10 µg/ml) and stimulated by 5 ng/ml *Escherichia coli* LPS (strain 026:B6; Difco Lab., Detroit, MI, USA) for 2 hours. Non-stimulated samples (added only peptide solution or 0.15 M NaCl) and positive control (blood + LPS) were incubated in parallel. All the experiments were performed at a gentle agitation (180 rpm) at 37 °C. LPS stimulation was terminated by adding Na₂EDTA (Merck, Darmstadt, Germany) at a final concentration of 5 mM to dissociate platelet-monocyte complexes.

### Quantitication of Monocyte TF Activity:

For TF activity assay, mononuclear cells (MNCs) were isolated from blood aliquots by density centrifugation using Lymphoprep (Axis-Shield, Norway) according to manufacturer instructions. The cell pellet was resuspended in 0.15 M NaCl and TF was measured using a two-stage clotting assay based on the ability of TF to accelerate the activation of factor X by factor VIIa as described by Østerud (1992). Cell counts were performed on a Sysmex K1000 (TOA Medical Electronics Co Ltd) in a whole blood system and after isolation of MNCs. Cell viability was assessed by using a trypan blue dye exclusion assay according to manufacturer instructions (Invitrogen Corp).

Figure 13 shows that P17 HC and P 17 HC-Br have potent anti-TF activities, inhibiting LPS induced TF response at low µM concentrations.

### Example 9

### Antibacterial killing assay

*E. coli* (ATCC 25922) was grown overnight in Mueller Hinton Broth (MHB) at 22 °C. The peptide P17 HC-Br and bacteria were mixed in sterile tubes to obtain the desired peptide concentration (0.78 and 1.56 µM) and final bacterial concentration of approximately 1 x 10⁶ colony forming units (cfu) per ml. The mixture was incubated at 22 °C with continuing orbital shaking. As negative and positive control the bacteria were added medium or Cecropin P1 (0.78 µM), respectively. At regular time intervals, aliquots of the mixture were removed, serially diluted in MHB, and plated in duplicate on MH agar. The agar plates were incubated overnight at 22 °C and colony forming units were counted. After 5 minutes of treatment with 0.78 µM P17 HC-Br, colony forming units were reduced by approximately 10⁴, and after 2 hours, no cfu were observed (figure 14).

### Example 10

### Wound healing

Substrate-specific allosteric inhibition of the proteasome activity may be tested by demonstrating that the polypeptides of the present invention stimulates angiogenesis and inhibits inflammatory responses by specifically blocking degradation of IκBα and HIF-1α by the proteasome. This will indicate the potensial of the polypeptides of the present invention in treating wounds. The ability of the polypeptides of the present invention to modulate wound healing may further be studied by topical application on wounds:
1) A linear incision of a part the skin of a mouse may be executed thus creating a wound. A composition comprising the polypeptides of the present invention may be applied to the wound. The same composition without the polypeptides of the present invention may serve as a control. After 1, 3 and 7 days the tensile strength of the treated skin may be tested and compared to the control.
2) A skin wound in a mammal is divided in two parts. A composition comprising the polypeptides of the present invention may be applied to one part, while a control composition without the polypeptides of the present invention may be applied to the other part. Photographs may be taken on day 1, 3, 7 after start of treatment, and healing of the sample and control is compared.

### Example 11

### Ulcers

Ulcers manifest as lesions or sore in the skin or mucous membrane resulting from a successive disintegration of surface epithelial tissues that make them distinctive from wounds. The ulcer might be superficial, but in most cases the ulcer extend into deeper layer creating a crater surrounded by defined edges, and are typically very painful. The etiologies of the different ulcers are different, where faulty blood circulation, infection, nerve damage, trauma or cancers might be inductive. Examples of ulcers are diabetic ulcers, plantar ulcers, peptic ulcers, decubitus ulcers, venous ulcer, ischemic ulcer, cancerous ulcers, aphthous ulcers, sublingual ulcers, and atonic ulcers.
A composition comprising the polypeptides of the present invention may be applied to a ulcer in an individual. The same composition without the polypeptides of the present invention, serving as control, may be applied to another ulcer in the same individual. The curative or ameliorating effects of the polypeptides of the present invention on ulcers may be demonstrated by comparing the ulcers at day 1, 3, 7 after start of treatment.

### Example 12

### Antiviral activity

To identify the antiviral activity of the polypeptides of the present invention, they may be tested against Herpes simplex virus (HSV), cytomegalovirus (CMV), adenovirus and enterovirus. These groups include both enveloped and naked viruses and also viruses associated with risk for the development of resistance. A MTT viability assay (Mosmann, 1983) may be used to evaluate the antiviral inhibition and the lytic activity of the viruses (CPE/MTT).

Having now fully described the present invention in some detail by way of illustration and example for purpose of clarity of understanding, it will be obvious to one of ordinary skill in the art that same can be performed by modifying or changing the invention by with a wide and equivalent range of conditions, formulations and other parameters thereof within the scope of the appended claims.

### REFERENCES

Altschul, S.F., Madden, T.L., Schaffer, A.A., Zhang, J., Zhang, Z., Miller, W. and Lipman, D.J. 1997. Gapped BLAST and PSI-BLAST: a new generation of protein database search programs, Nucleic Acids Res. 25, 3389-3402.
Andersson, L., Bohlin, L., Iorizzi, M., Riccio, R., Minale, L., and Moreno-López, W. 1989. Biological activity of some saponins and saponin-like compounds from starfish and brittle-stars. Toxicon 27, 179-188.
Bax, R., Mullan, N., and Verhoef, J. 2000. The millennium bugs - the need for and development of new antibacterials. Int. J. Antimicrob. Agents 16, 51-59.
Beauregard, K.A., Truong, N.T., Zhang, H.Y., Lin, W.Y., and Beck, G. 2001. The detection and isolation of a novel antimicrobial peptide from the echinoderm, Cucumaria frondosa. Adv. Exp. Med. Biol. 484, 55-62.
Brahmachary, M., Krishnan, S.P., Koh, J.L., Khan, A.M., Seah, S.H., Tan, T.W., Brusic, V. and Bajic, V.B. 2004. ANTIMIC: a database of antimicrobial sequences, Nucleic Acids Res. 32, 586-589.
Bryan, P.J., McClintock, J.B., Watts, S.A., Marion, K.R., and Hopkins, T.S., 1994. Antimicrobial activity of ethanolic extracts of echinoderms from the northern Gulf of Mexico. In: "Echinoderms through time" (B. David, A. Guille, J.-P. Feral, and M. Roux, Eds.), pp. 17-23, Balkema, Rotterdam.
Canicatti, C., and Roch, P. 1989. Studies on Holothuria polii (Echinodermata) antibacterial proteins. I. Evidence for and activity of a coelomocyte lysozyme. Experientia 45, 756-759.
De Vries, D.J., and Hall, M.R. 1994. Marine biodiversity as a source of chemical diversity. Drug Dev. Res. 33, 161-173.
Devine, D. A. and Hancock, R. E. 2002. Cationic peptides: distribution and mechanisms of resistance, Curr. Pharm. Des. 8, 703-714.
Epand, R.M. and Vogel, H.J. 1999. Diversity of antimicrobial peptides and their mechanisms of action, Biochim. Biophys. Acta - Biomem. 1462, 11-28.
Hancock, R.E. and Diamond, G. 2000. The role of cationic antimicrobial peptides in innate host defences, Trends Microbiol. 8, 402-410.
Hancock, R.E., and Rozek, A. 2002. Role of membranes in the activities of antimicrobial cationic peptides. FEMS Microbiol. Lett. 206, 143-149.
Haug, T., Kjuul, A.K., Stensvåg, K., Sandsdalen, E., and Styrvold, O.B. 2002a. Antibacterial activity in four marine crustacean decapods. Fish Shellfish Immunol. 12, 371-385.
Haug, T., Kjuul, A.K., Styrvold, O.B., Sandsdalen, E., Olsen, M.O., and Stensvag, K. 2002b. Antibacterial activity in Strongylocentrotus droebachiensis (Echinoidea), Cucumaria frondosa (Holothuroidea), and Asterias rubens (Asteroidea). J. Invertebr. Pathol. 81, 94-102.
Iorizzi, M., Bryan, P., McClintock, J., Minale, L., Palagiano, E., Maurelli, S., Riccio, R., and Zollo, F. 1995. Chemical and biological investigation of the polar constituents of the starfish Luidia clathrata, collected in the Gulf of Mexico. J. Nat. Prod. 58, 653-671.
Leonard, L.A., Strandberg, J.D., and Winkelstein, J.A. 1990. Complement-like activity in the sea star, Asterias forbesi. Dev. Comp. Immunol. 14, 19-30.
McGuffin, L.J., Bryson, K. and Jones, D.T. 2000. The PSIPRED protein structure prediction server, Bioinformatics. 16, 404-405.
Mor, A. 2000. Peptide-based antibiotics: A potential answer to raging antimicrobial resistance. Drug. Dev. Res. 50, 440-447.
Mosmann, T. 1983. Rapid colorimetric assay for cellular growth and survival: application to proliferation and cytotoxicity assays. J. Immunol. Methods 65, 55-63.
Multerer K.A. and Smith L.C. 2004. Two cDNAs from the purple sea urchin, Strongylocentrotus purpuratus, encoding mosaic proteins with domains found in factor H, factor I, and complement components C6 and C7. Immunogenetics. 56, 89-106.
Nilsen, I.W., Øverbø, K., Sandsdalen, E., Sandaker, E., Sletten, K., and Myrnes, B. 1999. Protein purification and gene isolation of chlamysin, a cold-active lysozyme-like enzyme with antibacterial activity. FEBS Lett. 464, 153-158.
Rinehart Jr., K.L., Shaw, P.D., Shield, L.S., Gloer, J.B., Harbour, G.C., Koker, M.E.S., Samain, D., Schwartz, R.E., Tymiak, A.A., Weller, D.L., Carter, G.T., Munro, M.H.G., Hughes Jr., R.G., Renis, H.E., Swynenberg, E.B., Stringfellow, D.A., Vavra, J.J., Coats, J.H., Zurenko, G.E., Kuentzel, S.L., Li, L.H., Bakus, G.J., Brusca, R.C., Craft, L.L., Young, D.N., and Connor, J.L. 1981. Marine natural products as sources of antiviral, antimicrobial, and antineoplastic agents. Pure & Appl. Chem. 53, 795-817.
Service, M., and Wardlaw, A.C. 1984. Echinochrome-A as a bactericidal substance in the coelomic fluid of Echinus esculentus (L.). Comp. Biochem. Physiol. B 79, 161-165.
Stabili, L., and Pagliara, P. 1994. Antibacterial protection in Marthasterias glacialis eggs - characterization of lysozyme-like activity. Comp. Biochem. Physiol. B 109, 709-713.
Tang, Y. Q., Yuan, J., Osapay, G., Osapay, K., Tran, D., Miller, C. J., Ouellette, A. J. and Selsted, M. E. 1999. A cyclic antimicrobial peptide produced in primate leukocytes by the ligation of two truncated alpha-defensins, Science. 286, 498-502.
Vesterlund, S., Paltta, J., Laukova, A., Karp, M. and Ouwehand, A.C. 2004. Rapid screening method for detection of antimicrobial substances. J. Microbiol. Meth. 57,23-31.
Wang, Z. and Wang, G. 2004. APD: the Antimicrobial Peptide Database, Nucleic Acids Res. 32, 590-592.
Wang, Z.M., Liu,C., and Dziarski, R. 2000. Chemokines are the main proinflamnatory mediators in human monocytes activated by Staphylococcus aureus, peptidoglycan and endotoxin. J. Biol. Chem. 275, 20260-20267.
Østerud B. 1992. Platelet activating factor enhancement of lipopolysaccharlde-induced tissue factor activity in monocytes: requirement of platelets and granulocytes. J Leukoc Biol. 51, 462-465.

**Table 1: Data on amino acids commonly found in peptides/proteins.**

| **Amino acid** | **One-letter symbol** | **Hydrophobic (nonpolar)** | **Polar, uncharged** | **Polar, charged** | **Other characteristics** |
|---|---|---|---|---|---|
| **Glycine** | G | | G | | |
| **Alanine** | A | A | | | |
| **Serine** | S | | S | | |
| **Proline** | P | P | | | Heterocyclic |
| **Valine** | V | V | | | |
| **Threonine** | T | | T | | |
| **Cysteine** | C | | C | | Sulfur-containing |
| **Leucine** | L | L | | | |
| **Isoleucine** | I | I | | | |
| **Asparagine** | N | | N | | |
| **Aspartic acid** | D | | | D (acidic) | |
| **Lysine** | K | | | K (basic) | |
| **Glutamine** | Q | | Q | | |
| **Glutamic acid** | E | | | E (acidic) | |
| **Methionine** | M | M | | | Sulfur-containing |
| **Histidine** | H | | | H (basic) | |
| **Phenylalanine** | F | F | | | Aromatic |
| **Arginine** | R | | | R (basic) | |
| **Tyrosine** | Y | | Y | | Aromatic |
| **Tryptophan** | W | W | | | Heterocyclic |

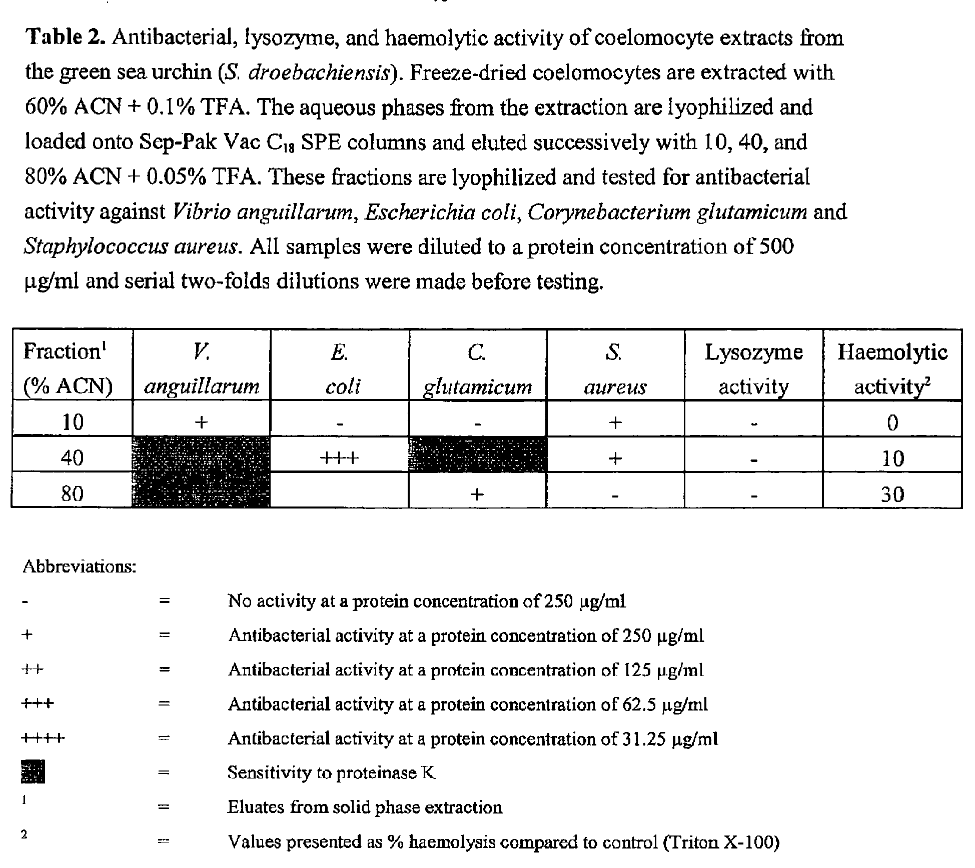

**Table 3. Amino acid composition of P 17 and P20, antimicrobial peptides isolated from S. droebachiensis coelomocytes. Values shown are in molecular percentage.**

| Amino acid | Composition (mol%) of | |
|---|---|---|
| | P17 | P20 |
| Asx¹ | 8.1 | 7.9 |
| Thr | 5.1 | 4.2 |
| Ser | 8.1 | 6.5 |
| Glx¹ | 8.8 | 5.9 |
| Pro | 5.5 | 2.8 |
| Gly | 14.3 | 14.6 |
| Ala | 14.2 | 13.5 |
| Val | 4.7 | 7.8 |
| Cys² | - | - |
| Met² | - | - |
| Ile | 2.0 | 4.6 |
| Leu | 6.3 | 12.5 |
| Tyr | - | - |
| Phe | 5.2 | 4.3 |
| Lys | 8.4 | 5.3 |
| His | 4.3 | 4.5 |
| Trp² | - | - |
| Arg | 4.7 | 5.6 |

| | | |
|---|---|---|
| ¹ Asx = Asp + Asn; Glx = Glu + Gln. ² Not determined due to destruction during acid hydrolysis. | | |

**Table 4. Susceptibility of the test strains to antibacterial peptides isolated from S. droebachiensis coelomocytes. Minimal inhibitory conentration (MIC) was determined as the lowest concentration of peptide causing an optical density less than 50% of the growth control. Polymyxin B, Lactoferricin B, Cecropin P1, and Cecropin B were used as reference peptides.**

| Peptide | Minimal inhibitory concentration (µM) | | | |
|---|---|---|---|---|
| | *L. anguillarum* | *E. coli* | *C. glutamicum* | *S. aureus* |
| P17 | 2.5 | 1.3 | 1.3 | 2.5 |
| P19 | 2.5 | 5.0 | 2.5 | 2.5 |
| P20 | 2.5 | 2.5 | 1.3 | 5.0 |
| Polymyxin B | 0.8 | 0.8 | 1.6 | 6.3 |
| Lactoferricin B | 3.1 | 3.1 | 1.6 | 6.3 |
| Cecropin P1 | 1.2 | 1.2 | 1.6 | 12.5 |
| Cecropin B | 0.6 | 0.5 | 1.0 | 9.4 |

**Table 5. Edman degradation amino acid sequencing of the antimicrobial peptides P17, P18, P19 and P20 isolated from sea urchin (S. droebachiensis) coelomocytes. P17 was reduced and alkylated before sequencing. The most probable amino acid is listed first in each cycle, and the identical amino acid sequence GIHAGQ are shown in bold.**

| **CYCLE** | **P17-alk.** | **P18** | **P19¹** | **P20** |
|---|---|---|---|---|
| **1** | GD | GDS | SD | SDH |
| **2** | L | L | L | L |
| **3** | FR | GRF | FR | FR |
| **4** | GK | SKH | AS | SAH |
| **5** | AK | KI | IR | RI |
| **6** | TC | FT | T | T |
| **7** | AF | KA | AV | VA |
| **8** | AH | KG | GH | HG |
| **9** | AK | AN | AN | NA |
| **10** | VH | FHV | HV | VH |
| **11** | SA | HA | GA | GA |
| **12** | HL | KL | NL | NL |
| **13** | A | VA | A | A |
| **14** | V | S | V | V |
| **15** | K | H | X | RK |
| **16** | S | V | X | K |
| **17** | G | V | G | G |
| **18** | I | R | I | I |
| **19** | H | Q | H | H |
| **20** | A | G | A | A |
| **21** | G | I | G | G |
| **22** | Q | H | Q | Q |
| **23** | R | A | G | G |
| **24** | G | G | VH | V |
| **25** | C | Q | X | X |
| **26** | S | S | S | SH |
| **27** | A | V | G | G |
| **28** | L | X | X | L |
| **29** | G | S | G | G |
| **30** | | G | | L |
| **31** | | V | | |
| **32** | | G | | |
| **33** | | L | | |

| | | | | |
|---|---|---|---|---|
| ¹ Due to an error during this sequencing, Lysine (K), if present, could not be detected. | | | | |

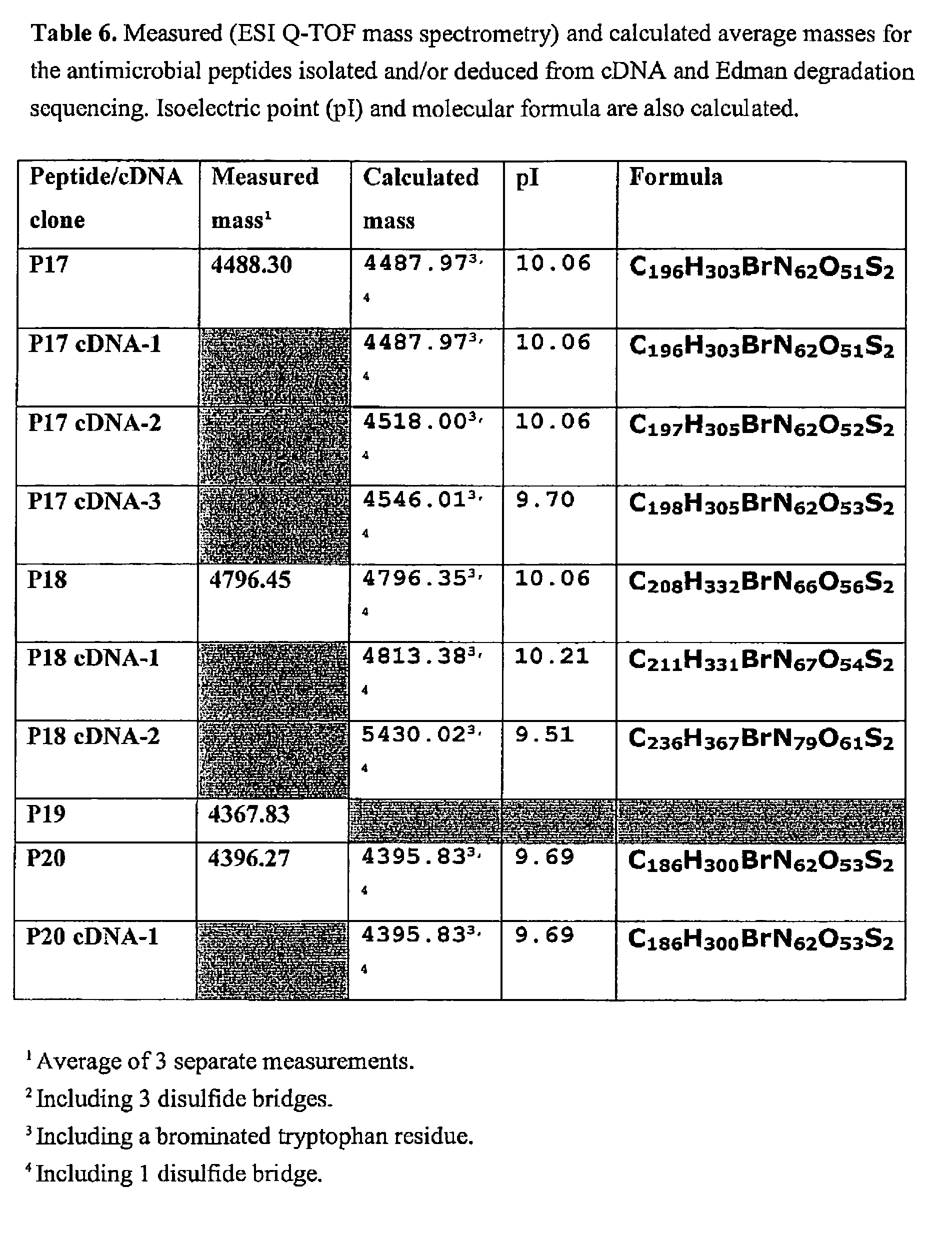

**Table 7 Overview of the residues in the formulas of the purified peptides P 17 - 20 (and the amino acids deduced from cDNA sequences related to these peptides).**

| Peptide | Hydrophobic nonpolar | Polar uncharged | Polar charged Basic | Polar charged Acidic | Modified or unknown residues |
|---|---|---|---|---|---|
| P17 | A2, A3 | A1 | A4,A5 | | |
| Heavy | A7 | A6 | A8,A9 | | |
| chain | A10 | A11 | A12 | | |
| (3) | A13,A14 | A16,A17 | A15 | | |
| | A18 | A21,A22 | A19 | | |
| | A20 | A24,A25,A26 | A23 | | |
| | A27,A28 | | | | |
| | A30 | A29 | | | |
| Light | A32 | A34 | A33 | A31 | |
| chain | A35 | A36 | | | |
| (4) | A37, | | | | |
| | A38,A39 | | A40 | | |
| | A41, A42 | | | | |
| P20 | A2, A3 | A1 | A5 | | |
| Heavy | A7 | A4 | | | |
| chain | A10 | A6 | A8, | | |
| | A13,A14 | A9 | | | |
| (5) | A18 | A11,A12 | A15,A16 | | |
| | A20 | A17 | A19 | | |
| | A24 | A21,A22A23 | | | |
| | A28 | A25,A26,A27 | | | |
| | A30 | A29 | | | |
| Light | A32 | | A33 | A31 | |
| chain | A34,A35 | A36 | | | |
| (6) | A37 | | | | |
| | A39 | A38 | | | |
| | A41,A42 | | A40 | | |
| P18 | A2,A6 | A1 | A5 | | |
| Heavy | A9,A10 | A3,A4 | A7,A8 | | |
| chain | A13 | A14 | A11,A12 | | |
| (7) | A16,A17 | A19,A20 | A15 | | |
| | A21 | | A18 | | |
| | A23 | A24,A25,A26 | A22 | | |
| | A27 | A28,A29,A30 | | | |
| | A31 | | | | |
| | A33 A32 | | | | |
| Light | A35 | A37 | A36 | A34 | |
| Chain | A38 | A39 | A43 | | |
| (8) | A40 | A41 | | | |
| | A42 | | | | |
| | A44,A45 | | | | |
| P19 | A2, A3 | A1 | A5 | | |
| Heavy | A7 | A4 | | | |
| chain | A10 | A6 | A8 | | |
| (9) | A13,A14 | A9 | | | |
| | A18 | A11,A12 | | | |
| | A20 | A17 | A19 | | A15,A16 |
| | A24 | A21,A22,A23 | | | |
| | | A25,A26,A27 | | | A28 |
| | | A29 | | | A30 |
| Light | A32 | | A33 | A31 | |
| Chain | A34,A35 | A36 | A40 | | |
| (10) | A37 | A38 | | | |
| | A39 | | | | |
| | A41,A42 | | | | |

**Table 8: Overview of the natural peptide P17 and synthesised fragments thereof.**

| Peptide/amino acid | Sequence | Calc. Mw^{a} | Obs. Mw^{a} | N^{b} | C^{c} | pI |
|---|---|---|---|---|---|---|
| P17 | | 4488.0 | 4488.3 | 42 | +5 | 10.06 |
| P17 HC^{d} | GWFKKTFHKVSHAVKSGIHAGQRGCSALGF | 3242.8 | 3240.9 | 30 | +5 | 10.48 |
| P17 HC (1-20) | GWFKKTFHKVSHAVKSGIHA | 2265.6 | 2265.8 | 20 | +4 | 10.48 |
| P17 HC (1-10) | GWFKKTFHKV | 1277.5 | 1277.7 | 10 | +3 | 10.30 |
| P17 HC (1-5) | GWFKK | 664.8 | 665.2 | 5 | +2 | 10.00 |
| P17 HC (11-20) | SHAVKSGIHA | 1006.1 | 1004.6 | 10 | +1 | 8.51 |
| P17 HC (21-30) | GQRGCSALGF | 995.1 | 993.6 | 10 | +1 | 8.25 |
| | | | | | | |
| P17 HG-Br | G*W*FKKTFHKVSHAVKSGIHAGQRGCSALGF | 3321.7 | 3320.3 | 30 | +5 | 10.48 |
| P17 HC-Br (1-20) | G*W*FKKTFHKVSHAVKSGIHA | 2344.5 | 2346.4 | 20 | +4 | 10.48 |
| P17 HC-Br (1-10) | G*W*FKKTFHKV | 1356.4 | 1357.4 | 10 | +3 | 10.30 |
| P17 HC-Br (1-5) | G*W*FKK | 743.7 | 743.4 | 5 | +2 | 10.00 |
| | | | | | | |
| P17 LC^{f} | DLRGACAAAHAL | 1168.3 | 1168.3 | 12 | 0 | 6.74 |
| | | | | | | |
| 5-D/L-Br-Trp | *W* | 283.2 | Nd^{g} | 1 | 0 | Nd |
| 6-D/L-Br-Trp | 6-Br-W | 283.2 | Nd | 1 | 0 | Nd |

| | | | | | | |
|---|---|---|---|---|---|---|
| ^{a} Theoretical and observed (MS) average molecular weights. ^{b} N: Number of amino acids in the peptides. ^{c} C: Net positive charge, ^{d} HC: Heavy chain of P17. ^{c} *W*: 5-D/L-Bromotryptophan. ^{f} LC: Light chain of 17. ^{g} Nd: Not determined. | | | | | | |

**Table 9. Minimal inhibitory concentration (µM) of synthesised P17-derivates against Gram-positive and Gram-negative bacterial strains at (a) 20°C and (b) 37°C. MIC was determined as the lowest concentration of peptide causing and optical density less than 50 % of the growth control.**

| a) | | | | |
|---|---|---|---|---|
| Peptide | *E. coli* | *L. anguillarum* | *C.glutamicum* | *S*. *aureus* |
| P17 HC-Br | 1.56 | 0.78 | 0.78 | 1.56 |
| P17 HC | 1.56 | 0.78 | 0.39 | 3.12 |
| P17 LC | >100 | >100 | >100 | >100 |

| b) | | | | |
|---|---|---|---|---|
| Peptide | *E. coli* | *P. aeruginosa* | *C.glutamicum* | *S. aureus* |
| P17 HC-Br | 1.56 | 3.12 | 0.39 | 6.25 |
| P17 HC-Br (1-20) | 6.25 | 6.25 | Nt | 100 |
| P17 HC-Br (1-10) | 25 | >50 | Nt | >100 |
| P17 HC-Br (1-5) | 25 | >50 | Nt | >100 |
| P17 HC | 1.56 | 3.12 | 0.78 | 25 |
| P17 HC (1-20) | 6.25 | 6.25 | 1.56 | 100 |
| P17 HC (1-10) | 100 | 100 | 12.5 | >100 |
| P17 HC (1-5) | >100 | >100 | >100 | >100 |
| P17 HC (11-20) | >100 | >100 | >100 | >100 |
| P17 HC (21-30) | >100 | >100 | >100 | >100 |
| P17 LC | >100 | >100 | >100 | >100 |

**Table 10. Minimal inhibitory concentrations (µM) of P17, P17 HC-Br and P17 HC (1-20) against human pathogenic (clinical) bacterial strains. MIC was determined as the lowest concentration of peptide causing and optical density less than 50 % of the growth control. Results for some non-clinical isolates are included for comparison.**

| | P17 HC-Br | P17 HC | P17 HC (1-20) | Polymyxin B |
|---|---|---|---|---|
| Clinical isolates | | | | |
| Escherichia coli, K15-58 | 3.12 | 1.56 | 6.25 | 3.12 |
| Klebsiella K15-72 | 3.12 | 3.12 | >50 | 6.25 |
| Acinetobacter baumannilii, K15-9 | 1.56 | 3.12 | 6.25 | 0.78 |
| Citrobacter freundii, K15-75 | 1.56 | 0.78 | 12.5 | 0.78 |
| Salmonella K14-40 | 6.25 | 6.25 | 50 | 3.12 |
| Bacillus spp K13-61 | 6.25 | 6.25 | 50 | Nt¹ |
| Pseudomonas K15-60 | 3.12 | 3.12 | 12.5 | 3.12 |
| Staphylococcus aureus (MRSA), K15-64 | 6.25 | 6.25 | >50 | Nt |
| Enterococcus faecium (GREF), K9-28 | 0.78 | 0.39 | 6.25 | Nt |
| | | | | |

| Non-clinical isolates | | | | |
|---|---|---|---|---|
| Escherichia coli, ATCC 25922 | 6.25 | 6.25 | Nt | Nt |
| Pseudomonas aeruginosa ATCC 27853 | 6.25 | 6.25 | Nt | Nt |
| Corynebacterium glutamicum, ATCC 13032 | 0.78 | 0.78 | 3.12 | Nt |
| Staphylococcus aureus, ATCC 9144 | 12.5 | 12.5 | >50 | Nt |

| | | | | |
|---|---|---|---|---|
| ¹ Nt = Not tested. | | | | |

**Table 11. Minimal inhibitory concentration (MIC, µM) of synthesised P17-derivates against various fungal strains. MIC was determined as the lowest concentration of peptide resulting in more than 50% inhibition of visible growth.**

| Peptide | Yeast | | | Filamentous fungi | | |
|---|---|---|---|---|---|---|
| | *Saccharomyces cerevisiae* | *Rhodotorula* sp. | *Candida albicans* | *Botrytis cinerea* | *Fusarium* sp. | *Penicillum rogveforti* |
| P17 HC-Br | 6.25 | 3.12 | 6.25 | 25 | 1.58 | 6.25 |
| P17 HC-Br (1-20) | 12.5 | Nt¹ | 6.25 | 50 | Nt | Nt |
| P17 HC-Br (1-10) | 25 | Nt | 25 | 50 | Nt | Nt |
| P17 HC-Br (1-5) | >100 | Nt | >100 | >100 | Nt | Nt |
| P17 HC | 6.25 | 3.12 | 6.25 | 50 | 1.56 | 6.25 |
| P17 HC (1-20) | 12.5 | Nt | 6.25 | 100 | Nt | Nt |
| P17 HC (1-10) | 25 | Nt | 25 | 100 | Nt | Nt |
| P17 HC (1-5) | >100 | Nt | >100 | >100 | Nt | Nt |
| P17 HC (11-20) | >100 | Nt | >100 | >100 | Nt | Nt |
| P17 HC (21-30) | >100 | Nt | >100 | >100 | Nt | Nt |
| P17 LC | >100 | Nt | >100 | >100 | Nt | >100 |
| Cecropin B | 3.12 | 3.12 | 6.25 | 25 | 1.56 | 3.12 |

| | | | | | | |
|---|---|---|---|---|---|---|
| ¹ Nt: Not tested. | | | | | | |

**Table 12a Antitumoral activities (IC₅₀, µM) of P17 HC, P17 HC-Br and P17 LC. The maximum concentration of the peptides tested was 100 µg/ml.**

| | IC₅₀ | | | | IC₅₀ Ratio^{a} |
|---|---|---|---|---|---|
| Peptide/amino acid | FemX | MT-1 | HT-29 | MRC-5 | |
| P17HC | 9 | *^{b} | -^{c} | - | >3.4 |
| P17 HC-Br | 6 | 20 | - | - | >5.0 |
| P17LC | - | - | - | - | - |

| | | | | | |
|---|---|---|---|---|---|
| ^{a} IC₅₀ ratio MRC-5 (Fibroblasts)/FemX. ^{b} The symbol "*" denotes 30-50% killing at the highest concentration tested. ^{c} The symbol "-" denotes no activity within the concentration range tested. | | | | | |

**Table 12b. Antitumoral activities (IC₅₀, µM) of natural P17, shorter synthetic derivatives of P17, and brominated tryptophans. The cells were treated with peptides/amino acids diluted in serum-free RPMI-1640 medium. The maximum concentration of the compounds tested was 200 µg/ml.**

| | IC₅₀ | | | | IC₅₀ Ratio^{a} |
|---|---|---|---|---|---|
| Peptide/amino acid | HT-29 | MCF-7 | A2058 | MRC-5 | |
| P17 | Nd^{b} | Nd | 28 | Nd | - |
| P17 HC | 52 | 13 | 9 | 53 | 5.9 |
| P17 HC (1-20) | -^{c} | - | - | - | - |
| P17 HC (1-10) | - | - | - | - | - |
| P17 HC (1-5) | - | - | - | - | - |
| P17 HC (11-20) | - | 103 | *^{d} | * | - |
| P17 HC (21-30) | - | - | - | * | - |
| P17 HC-Br | 76 | 12 | 15 | 50 | 3.3 |
| P17LC | - | - | - | - | - |
| 5-D/L-Br-Trp | * | - | - | * | - |
| 6-D/L-Br-Trp | * | * | 655 | 584 | 0.9 |

| | | | | | |
|---|---|---|---|---|---|
| ^{a} IC₅₀ ratio MRC-5 (Fibroblasts)/A2058. ^{b} Nd: Not determined. ^{c} The symbol "-" denotes no activity within the concentration range tested. ^{d} The symbol "*" denotes 30-50% killing at the highest concentration tested. | | | | | |

**Table 12c. Antitumoral activities (IC₅₀, µM) of synthetic derivatives of P17 and brominated tryptophans. The cells were treated with peptides/amino acids diluted in RPMI-1640 medium containing 5% FBS. The maximum concentration of the compounds tested was 200 µg/ml.**

| | IC₅₀ | | | | IC₅₀ Ratio^{a} |
|---|---|---|---|---|---|
| Peptide/amino acid | HT-29 | MCF-7 | A2058 | MRC-5 | |
| P17 HC | 52 | 18 | 7 | 53 | 7.6 |
| P17 HC (1-20) | -^{b} | - | *^{c} | - | - |
| P17 HC (1-10) | - | - | - | - | - |
| P17 HC (1-5) | - | - | - | - | - |
| P17 HC (11-20) | * | - | - | - | - |
| P17 HC (21-30) | - | - | - | - | - |
| P17 HC-Br | 59 | 15 | 14 | 38 | 2.7 |
| P17 LC | - | - | - | - | - |
| 5-D/L-Br-Tip | - | - | - | * | - |
| 6-D/L-Br-Trp | * | * | * | * | - |

| | | | | | |
|---|---|---|---|---|---|
| ^{a} IC₅₀ ratio MRC-5 (Fibroblasts)/A2058. ^{b} The symbol "-" denotes no activity within the concentration range tested. ^{c} The symbol "*" denotes 30-50% killing at the highest concentration tested. | | | | | |

**Table 12d. Antitumoral activities (IC₅₀, µM) of synthetic derivatives of P 17 against murine tumor cell lines CT-26 and A20. The cells were treated with peptides diluted in serum-free RPMI-1640 medium for 4 hours. The maximum concentration of the compounds tested was 200 µg/ml.**

| | IC₅₀ | |
|---|---|---|
| | CT-26 | A20 |
| Peptide | | |
| P 17 HC | 20.4 | 3.9 |
| P17 HC-Br | 33.1 | 24.1 |
| P17 HC-Br (1-20) | 59.7 | -^{a} |
| P17 HC-Br (1-10) | - | - |
| P17 HC-Br (1-5) | - | - |

| | | |
|---|---|---|
| ^{a} The symbol "-" denotes no activity within the concentration range tested. | | |

**Table 13. Antiinflammatory (anti- TNFα, EC₅₀, µM) and cytotoxic activities (TC₅₀, µM) of peptide fragments derived from P 17 and two brominated amino acids on THP-1 cells. The maximum concentration of the compounds tested (toxicity) against THP-1 cells was 800 µg/ml. EC₅₀ was defined as the concentration of test substance reducing the TNFα levels with 50% compared to control (only LPS), whereas TC₅₀ was defined as the concentration of test substance killing 50% of the THP-1 cells compared to control (Triton X-100).**

| Peptide/amino acid | Anti-TNFα EC₅₀(µM) | Toxicity to THP-1 cells TC₅₀(µM) | Therapeutic index^{a} TC₅₀/EC₅₀ |
|---|---|---|---|
| P17 HC | 2.8 | 38.0 | 13.6 |
| P17 HC (1-20) | 20.3 | -^{b} | >17.4 |
| P17 HC (1-10) | - | - | - |
| P17 HC (1-5) | - | - | - |
| P17 HC (11-20) | 358.7 | - | >2.2 |
| P17 HC (21-30) | - | - | - |
| P17 HC-Br | 1.1 | 51.1 | 46.5 |
| P 17 LC | - | - | - |
| 5-D/L-Br-Trp | - | Nt^{c} | - |
| 6-D/L-Br-Trp | - | Nt | - |

| | | | |
|---|---|---|---|
| ^{a} Ratio TC₅₀(THP-1)/EC₅₀(anti-TNFα). ^{b} The symbol "-" denotes no activity within the concentration range tested. ^{c} Nt: Not tested. | | | |

**Table 14. Minimal inhibitory concentration (MIC, µM) of synthesised P17-derivates causing more than 50% reduction in luminescence (a) or optical density (b) compared to the growth control, with or without co-incubation with 2.5 µM Aeromonas salmonicida LPS. Peptide (P) concentration ranged from 0.6 to 80 µM. Bioluminescence and optical density were registrated as counts per second (cps) and absorbance at 600 nm (OD₆₀₀), respectively.**

| a) | | | | | | |
|---|---|---|---|---|---|---|
| Peptide | *E. coli* | | *S. enterica* | | *S. aureus* | |
| | P | P + LPS | P | P + LPS | P | P + LPS |
| P17 HC-Br | 0.6 | 5 | 1.25 | 10 | 20 | 20 |
| P17 HC-Br (1-20) | 20 | 20 | 2.5 | 10 | - | - |
| P17 HC-Br (1-10) | - | - | 20 | 20 | - | - |
| P17 HC-Br (1-5) | - | - | - | - | - | - |
| P17 HC | 0.6 | 5 | 1.25 | 5 | 20 | 20 |
| P17 HC (1-20) | 20 | 40 | 2.5 | 5 | - | - |
| P17 HC (1-10) | - | - | - | - | - | - |
| P17 HC (1-5) | - | - | - | - | - | - |
| P17 HC (11-20) | - | - | - | - | - | - |
| P17 HC (21-30) | - | - | - | - | - | - |
| P17 LC | - | - | - | - | - | - |
| Cecropin P1 | 2.5 | 20 | 2.5 | 10 | 40 | - |

| b) | | | | | | |
|---|---|---|---|---|---|---|
| Peptide | *E. coli* | | *S. enterica* | | *S. aureus* | |
| | P | P + LPS | P | P + LPS | P | P + LPS |
| P17 HC-Br | 0.6 | 5 | 1.25 | 10 | 20 | 40 |
| P17 HC-Br (1-20) | 10 | 40 | 2.5 | 10 | - | - |
| P17 HC-Br (1-10) | - | - | 40 | 40 | - | - |
| P17 HC-Br (1-5) | - | - | - | - | - | - |
| P17 HC | 0.6 | 5 | 1.25 | 2.5 | 20 | 40 |
| P17 HC (1-20) | 40 | 80 | 2.5 | 10 | - | - |
| P17 HC (1-10) | - | - | - | - | - | - |
| P17 HC (1-5) | - | - | - | - | - | - |
| P17 HC (11-20) | - | - | - | - | - | - |
| P17 HC (21-30) | - | - | - | - | - | - |
| P17LC | - | - | - | - | - | - |
| Cecropin P1 | 2.5 | 20 | 2.5 | 10 | 80 | - |

## Claims

1. A polypeptide comprising one of the following amino acid sequences:
a) amino acid sequence SEQIDNO41;
b) amino acid residues 1-20 of SEQIDNO3;
c) amino acid residues 1-20 of SEQIDNO5;
d) amino acid residues 2-20 of SEQIDNO7;
wherein amino acid residue number 2 is tryptophan or brominated tryptophan;
said polypeptide having antimicrobial activity.

2. The polypeptide according to claim 1, comprising one of the following amino acid sequences:
a) SEQIDNO1;
b) SEQIDNO3;
c) SEQIDNO5;
d) SEQIDNO7.

3. The polypeptide according to any one of claims 1-2, wherein said polypeptide is selected from the group consisting of:
a) SEQIDNO1;
b) SEQIDNO3;
c) SEQIDNO5; and
d) SEQIDNO7.

4. Dimeric polypeptide, wherein the first monomer is the polypeptide according to any one of claims 1-3.

5. The dimeric polypeptide according to claim 4, wherein the second monomer comprises the amino acid sequence DLRxxxAxAHAL; x being any amino acid.

6. An isolated nucleic acid molecule, encoding the polypeptide according to any one of claims 1-3 or the dimeric polypeptide according to any one of claims 4-5.

7. A vector comprising the nucleic acid according to claim 6.

8. A suitable host cell comprising the vector of claim 7.

9. A method of producing the polypeptide according to any one of claims 1-3 or the dimeric polypeptide according to any one of claims 4-5, comprising cultivating the host cell of claim 8 and isolating the polypeptide or the dimeric polypeptide.

10. The polypeptide according to any one of claims 1-3, the dimeric polypeptide according to any one of claims 4-5, the nucleic acid according to claim 6 or the vector of claim 7 for medical use.

11. Use of the polypeptide according to any one of claims 1-3, the dimeric polypeptide according to any one of claims 4-5, the nucleic acid according to claim 6 or the vector of claim 7 for manufacturing a medicament for the treatment of fungus, infections, ulcers, wounds, cancer and/or inflammation in a patient in need thereof.

12. An antibody or antibody fragment that specifically binds with the polypeptide according to claim 3.

13. A pharmaceutical formulation comprising the polypeptide according to any one of claims 1-3, the dimeric polypeptide according to any one of claims 4-5, the nucleic acid according to claim 6 or the vector of claim 7; and a pharmaceutical acceptable vehicle.

## Patentansprüche

1. Polypeptid, umfassend eine der folgenden Aminosäuresequenzen:
a) Aminosäuresequenz SEQIDNO41;
b) Aminosäurereste 1-20 von SEQIDNO3;
c) Aminosäurereste 1-20 von SEQIDNO5;
d) Aminosäurereste 1-20 von SEQIDNO7;
wobei die Aminosäurerest-Nummer 2 Tryptophan oder bromiertes Tryptophan ist; wobei das Polypeptid eine antimikrobielle Aktivität aufweist.

2. Polypeptid nach Anspruch 1, umfassend eine der folgenden Aminosäuresequenzen:
a) SEQIDN01;
b) SEQIDNO3;
c) SEQIDNO5;
d) SEQIDNO7.

3. Polypeptid nach irgendeinem der Ansprüche 1-2, wobei das Polypeptid aus der Gruppe ausgewählt ist, bestehend aus:
a) SEQIDN01;
b) SEQIDNO3;
c) SEQIDNO5; und
d) SEQIDNO7.

4. Dimeres Polypeptid, wobei das erste Monomer das Polypeptid nach irgendeinem der Ansprüche 1-3 ist.

5. Dimeres Polypeptid nach Anspruch 4, wobei das zweite Monomer die Aminosäuresequenz DLRxxxAxAHAL umfasst; wobei x irgendeine Aminosäure ist.

6. Isoliertes Nukleinsäuremolekül, welches das Polypeptid nach irgendeinem der Ansprüche 1-3 oder das dimere Polypeptid nach irgendeinem der Ansprüche 4-5 kodiert.

7. Vektor, umfassend die Nukleinsäure nach Anspruch 6.

8. Geeignete Wirtszelle, umfassend den Vektor nach Anspruch 7.

9. Verfahren zur Herstellung des Polypeptids nach irgendeinem der Ansprüche 1-3 oder des dimeren Polypeptids nach irgendeinem der Ansprüche 4-5, umfassend die Kultivierung der Wirtszelle nach Anspruch 8 und Isolierung des Polypeptids oder des dimeren Polypeptids.

10. Polypeptid nach irgendeinem der Ansprüche 1-3, das dimere Polypeptid nach irgendeinem der Ansprüche 4-5, die Nukleinsäure nach Anspruch 6 oder der Vektor nach Anspruch 7 zur medizinischen Verwendung.

11. Verwendung des Polypeptids nach irgendeinem der Ansprüche 1-3, des dimeren Polypeptids nach irgendeinem der Ansprüche 4-5, der Nukleinsäure nach Anspruch 6 oder des Vektors nach Anspruch 7 zur Herstellung eines Medikaments zur Behandlung von Pilz, Infektionen, Ulcera, Wunden, Krebs und/oder Entzündung bei einem behandlungsbedürftigen Patienten.

12. Antikörper oder Antikörperfragment, welcher/welches spezifisch an das Polypeptid nach Anspruch 3 bindet.

13. Pharmazeutische Formulierung, umfassend das Polypeptid nach irgendeinem der Ansprüche 1-3, das dimere Polypeptid nach irgendeinem der Ansprüche 4-5, die Nukleinsäure nach Anspruch 6 oder den Vektor nach Anspruch 7; und ein pharmazeutisches annehmbares Vehikel.

## Revendications

1. Polypeptide comprenant l'une des séquences d'acide aminé suivantes:
a) la séquence d'acide aminé SEQIDNO41;
b) les résidus d'acide aminé 1-20 de SEQIDNO3;
c) les résidus d'acide aminé 1-20 de SEQIDNO5;
d) les résidus d'acide aminé 1-20 de SEQIDNO7;
dans lesquelles le numéro 2 des résidus d'acide aminé est le tryptophane ou le tryptophane bromé; ledit polypeptide présentant une activité antimicrobienne.

2. Polypeptide selon la revendication 1, comprenant l'une des séquences d'acide aminé suivantes:
a) SEQIDNO1;
b) SEQIDNO3;
c) SEQIDNO5;
d) SEQIDNO7;

3. Polypeptide selon l'une quelconque des revendications 1 à 2, dans lequel ledit polypeptide est choisi parmi le groupe constitué par:
a) SEQIDNO1;
b) SEQIDNO3;
c) SEQIDNO5; et
d) SEQIDNO7.

4. Polypeptide dimère, dans lequel le premier monomère est le polypeptide selon l'une quelconque des revendications 1 à 3.

5. Polypeptide dimère selon la revendication 4, dans lequel le deuxième monomère comprend la séquence d'acide aminé DLRxxxAxAHAL; x étant tout acide aminé.

6. Molécule d'acide nucléique isolée, codant le polypeptide selon l'une quelconque des revendications 1 à 3 ou le polypeptide dimère selon l'une quelconque des revendications 4 à 5.

7. Vecteur comprenant l'acide nucléique selon la revendication 6.

8. Cellule hôte comprenant le vecteur selon la revendication 7.

9. Procédé de production du polypeptide selon l'une quelconque des revendications 1 à 3 ou du polypeptide dimère selon l'une quelconque des revendications 4 à 5, comprenant la culture de la cellule hôte selon la revendication 8 et l'isolement du polypeptide ou le polypeptide dimère.

10. Polypeptide selon l'une quelconque des revendications 1 à 3, le polypeptide dimère selon l'une quelconque des revendications 4 à 5, l'acide nucléique selon la revendication 6 ou le vecteur selon la revendication 7 à l'usage médical.

11. Usage du polypeptide selon l'une quelconque des revendications 1 à 3, du polypeptide dimère selon l'une quelconque des revendications 4 à 5, de l'acide nucléique selon la revendication 6 ou du vecteur selon la revendication 7 pour la préparation d'un médicament pour le traitement des champignons, des infections, des ulcères, des plaies, du cancer et/ou d'une inflammation chez un patient le nécessitant.

12. Anticorps ou fragment d'anticorps qui se lie spécifiquement avec le polypeptide selon la revendication 3.

13. Formulation pharmaceutique comprenant le polypeptide selon l'une quelconque des revendications 1 à 3, le polypeptide dimère selon l'une quelconque des revendications 4 à 5, l'acide nucléique selon la revendication 6 ou le vecteur selon la revendication 7; et un véhicule pharmaceutiquement acceptable.
